# EUROPEAN PATENT APPLICATION

(11) **EP 4 487 793 A1**
(43) Date of publication of application: **08.01.2025**
(21) Application number: 23770622.1
(22) Date of filing: 09.03.2023
(51) Int. Cl.: A61B 17/135, A61B 17/132

(54) **HEMOSTASIS DEVICE**

(30) Priority: 18.03.2022 JP 2022044655
(71) Applicant: Terumo Kabushiki Kaisha, Tokyo 151-0072 (JP)
(72) Inventor: KAWAMURA, Tomoya, Fujinomiya-shi, Shizuoka 418-0015 (JP); OUCHI, Tatsuya, Fujinomiya-shi, Shizuoka 418-0015 (JP); HIDAKA, Yuna, Fujinomiya-shi, Shizuoka 418-0015 (JP)
(74) Representative: Casalonga
(86) International application number: PCT/JP2023/008987
(87) International publication number: WO 2023/176666

(57) **Abstract**

There is provided a hemostatic device capable of easily securing an injection member for injecting a fluid into an inflatable member to a cover member and preventing the hemostatic device and the inflatable member from being displaced or the like when the injection member is secured to the cover member.

An injection member 200 of a hemostatic device 10 includes a connector portion 210 and a tube member 220 coupled to an inflatable member 150, a securing member 230 configured to be capable of securing the injection member to a cover member 100 includes a main body 231 connecting the connector portion and the tube member, claw portions 233 and 234 extending in parallel with the main body toward the inflatable member, coupling portions 236 and 237 configured to be capable of inserting the cover member between the main body and the claw portions and coupling the main body and the claw portions, and a width w3 of the claw portions is smaller than a width w4 of the main body at a position where the claw portions overlap the main body in a plan view.

## Description

### Technical Field

The present invention relates to a hemostatic device.

### Background Art

As one of catheter procedures, there is known a procedure in which various medical elongated bodies are introduced into a blood vessel of a limb such as an arm or hand of a patient through a puncture site formed by puncturing the blood vessel to perform a diagnosis or a therapy at a lesion site. For example, Patent Literature 1 discloses a hemostatic device for stopping bleeding at a puncture site formed to enable access to a blood vessel (including a distal radial artery) running in a hand.

The hemostatic device of Patent Literature 1 includes: an injection member including an inflatable member that applies a compressive force to a puncture site formed on a hand of a patient, a securing member for securing a pressing member to the hand of the patient, a port (connector portion) configured to be capable of injecting a fluid for inflating the inflatable member, and a tube portion connecting the connector portion and an inner cavity of the inflatable member; and a tube securing portion located on the securing member and configured to be capable of securing the tube member.

### Citation List

### Patent Literature

Patent Literature 1: WO 2019/090104

### Summary of Invention

### Technical Problem

When hemostasis is performed at the puncture site formed on the hand of the patient using the hemostatic device of Patent Literature 1, an operator such as a doctor (hereinafter, referred to as the "operator") can prevent the inflatable member from being displaced from the puncture site formed on the hand of the patient by securing the hemostatic device using each band body in a state where the inflatable member is disposed at the puncture site formed on the hand of the patient and a peripheral portion thereof. Furthermore, the operator can suppress a movement of the tube member following a movement of the hand of the patient by securing the tube member to the tube securing portion.

However, the hemostatic device described in Patent Literature 1 may have the following problems.

In the hemostatic device of Patent Literature 1, a groove for securing the tube member is provided in the tube securing portion. Therefore, when the operator attaches and detaches the tube member to and from the tube securing portion, the operator needs to deform (for example, elastically deform) the tube securing portion in a direction of widening the groove by applying an external force to the tube member. At this time, a force required for the operator to deform the tube securing portion in the direction of widening the groove is determined by a mechanical configuration of the tube securing portion, but such a configuration is not designed in consideration of operability and convenience of the operator in some cases.

For example, in a case where a force required for the operator to attach and detach the tube member to and from the tube securing portion is large, there is a possibility that the external force (force applied by the operator) is transmitted to the inflatable member via the securing member provided with the tube securing portion so that the external force is unintentionally applied to the puncture site. Furthermore, there is a possibility that the inflatable member is displaced from the puncture site formed on the hand of the patient as the external force is unintentionally transmitted to the inflatable member when the external force is transmitted to the tube securing portion.

Furthermore, Patent Literature 1 discloses the hemostatic device having a structure in which the port enabling the injection of the fluid for inflating the inflatable member is directly coupled to the inflatable member.

The port directly coupled to the inflatable member is disposed so as to protrude from an outer surface side located opposite to a body surface of the cover member. Therefore, if the operator connects a fluid supply instrument such as a syringe to the port at the time of inflating the inflatable member, a pushing force at the time of connection is transmitted to the entire hemostatic device, and there is a possibility that the hemostatic device is displaced or the hemostatic device is disposed to be inclined. If the hemostatic device is displaced or disposed to be inclined as described above, there is a possibility that the inflatable member is displaced or a direction in which the puncture site is compressed by the inflatable member changes to an unintended direction.

The present invention has been made based on the above problems, and an object thereof is to provide a hemostatic device capable of easily securing an injection member for injecting a fluid into an inflatable member to a cover member and preventing the hemostatic device and the inflatable member from being displaced or the like when the injection member is secured to the cover member.

### Solution to Problem

A hemostatic device according to one mode of the present invention includes: a cover member configured to cover a puncture site formed on a patient; an inflatable member connected to the cover member and configured to compress the puncture site; an injection member connected to the inflatable member and configured to be capable of injecting a fluid into the inflatable member; and a securing member configured to be capable of securing the injection member to the cover member, wherein the injection member includes a connector portion and a tube member coupled to the inflatable member, the securing member includes a main body connecting the connector portion and the tube member, a claw portion extending in parallel with the main body toward the inflatable member, and a coupling portion configured to be capable of inserting the cover member between the main body and the claw portion and coupling the main body and the claw portion, and a width of the claw portion is smaller than a width of the main body at a position where the claw portion overlaps the main body in a plan view

A hemostatic device according to another mode of the present invention includes: a cover member configured to cover a puncture site formed on a patient; an inflatable member connected to the cover member and configured to compress the puncture site; an injection member connected to the inflatable member and configured to be capable of injecting a fluid into the inflatable member; and a securing member configured to be capable of securing the injection member to the cover member, wherein the injection member includes a connector portion and a tube member coupled to the inflatable member, the securing member includes a main body connecting the connector portion and the tube member, a claw portion extending in parallel with the main body toward the inflatable member, and a coupling portion configured to be capable of inserting the cover member between the main body and the claw portion and coupling the main body and the claw portion, the main body includes an inclined portion inclined toward the claw portion, and at least a part of the inclined portion is configured to be located at a position on a proximal side of a distal end of the claw portion in the main body.

### Advantageous Effects of Invention

In the hemostatic device according to one mode of the present invention, the injection member can be secured to the cover member by inserting the cover member between the main body and the claw portion in a state where the main body of the securing member connects the connector portion and the tube member. When inserting the cover member between the main body and the claw portion, an operator can insert the cover member between the main body and the claw portion by bringing the securing member close to the cover member along a direction substantially parallel with a plane direction of the cover member. Therefore, when the operator secures the injection member to the cover member, an unnecessary force can be prevented from being applied in a direction of pressing the hemostatic device against a body surface of the patient's body. Therefore, the hemostatic device can prevent the hemostatic device worn on the patient's body from being displaced or the inflatable member from being displaced when the injection member is secured to the cover member. Furthermore, for example, the cover member may be curved following a movement of the body in a state where the hemostatic device is worn on the patient. In the hemostatic device, the width of the claw portion is smaller than the width of the main body at the position where the claw portion overlaps the main body in the plan view. Therefore, in the hemostatic device, the cover member can be smoothly inserted between the main body and the claw portion even in a state where the cover member is curved following the movement of the patient.

In the hemostatic device according to another mode of the present invention, the injection member can be secured to the cover member by inserting the cover member between the main body and the claw portion in a state where the main body of the securing member connects the connector portion and the tube member. When inserting the cover member between the main body and the claw portion, an operator can insert the cover member between the main body and the claw portion by bringing the securing member close to the cover member along a direction substantially parallel with a plane direction of the cover member. Therefore, when the operator secures the injection member to the cover member, an unnecessary force can be prevented from being applied in a direction of pressing the hemostatic device against a body surface of the patient's body. Therefore, the hemostatic device can prevent the hemostatic device worn on the patient's body from being displaced or the inflatable member from being displaced when the injection member is secured to the cover member. Furthermore, the hemostatic device is configured such that the main body includes the inclined portion inclined toward the claw portion. Furthermore, at least a part of the inclined portion of the main body is configured to be located at the position on the proximal side of the distal end of the claw portion in the main body. Therefore, the hemostatic device described above is configured such that when the operator inserts the cover member between the main body and the claw portion, the inclined portion guides the cover member to a position closer to the coupling portion than the distal end of the claw portion. Therefore, when inserting the cover member between the main body and the claw portion, the operator can smoothly insert the cover member between the main body and the claw portion while adjusting a shape of the cover member by guiding the cover member along the inclined portion.

### Brief Description of Drawings

Fig. 1 is a view illustrating a hemostatic device according to a first embodiment, and is a plan view seen from an outer surface side of each band body.
Fig. 2 is a view illustrating the hemostatic device according to the first embodiment, and is a plan view seen from an inner surface side of each band body.
Fig. 3 is an enlarged view illustrating a part of the hemostatic device as seen from an outer surface side of a main body of a cover member.
Fig. 4 is an enlarged view illustrating a part of the hemostatic device as seen from an inner surface side of the main body of the cover member.
Fig. 5 is an enlarged view illustrating a part of the hemostatic device as seen from the outer surface side of the main body of the cover member.
Fig. 6 is a partial cross-sectional view of the hemostatic device taken along an arrow 6A-6A illustrated in Fig. 5, and is a view illustrating a state when an inflatable member inflates.
Fig. 7 is a partial cross-sectional view of the hemostatic device taken along an arrow 7A-7A illustrated in Fig. 5, and is a view illustrating a state when the inflatable member inflates.
Fig. 8 is a plan view of a support member.
Fig. 9 is a perspective view of the support member as seen from a lower end side.
Fig. 10 is a perspective view illustrating an inner face side of the support member.
Fig. 11 is a perspective view of an injection member.
Fig. 12 is a cross-sectional view of a main body of the injection member taken along an arrow 12A-12A illustrated in Fig. 11.
Fig. 13 is a side view of the injection member seen from a direction of an arrow 13A illustrated in Fig. 12.
Fig. 14 is a bottom view of the injection member seen from a direction of an arrow 14A illustrated in Fig. 12.
Fig. 15 is a view illustrating a state when the cover member is inserted between a main body and a claw portion of a securing member.
Fig. 16 is a view illustrating a state when the cover member is inserted between the main body and the claw portion of the securing member.
Fig. 17 is a view for describing an operational effect of the securing member.
Fig. 18 is a view for describing the operational effect of the securing member.
Fig. 19 is a view illustrating a hand (right hand) of a patient for which the hemostatic device according to the first embodiment is to be used.
Fig. 20 is a view briefly illustrating a use example of the hemostatic device according to the first embodiment.
Fig. 21 is a view briefly illustrating the use example of the hemostatic device according to the first embodiment.
Fig. 22 is a view briefly illustrating the use example of the hemostatic device according to the first embodiment.
Fig. 23 is a partial cross-sectional view taken along an arrow 23A-23A illustrated in Fig. 22.
Fig. 24 is a partial cross-sectional view taken along an arrow 24A-24A illustrated in Fig. 22.
Fig. 25 is a view illustrating a position where the injection member is secured.
Fig. 26 is a view illustrating the position where the injection member is secured.
Fig. 27 is a view illustrating the position where the injection member is secured.
Fig. 28 is a side view of an injection member according to a first modification.
Fig. 29 is a partial cross-sectional view taken along an arrow 29A-29A illustrated in Fig. 28.
Fig. 30 is a side view of an injection member according to a second modification.
Fig. 31 is a side view of an injection member according to a third modification.
Fig. 32 is a view briefly illustrating a use example of a hemostatic device according to a fourth modification.
Fig. 33 is a view briefly illustrating the use example of the hemostatic device according to the fourth modification.
Fig. 34 is a perspective view illustrating a securing member according to a second embodiment.
Fig. 35 is a side view of the securing member as seen from a direction of an arrow 35A illustrated in Fig. 34.
Fig. 36 is a view briefly illustrating a use example of the securing member according to the second embodiment.

### Description of Embodiments

Hereinafter, embodiments of the present invention will be described with reference to the accompanying drawings. The following description does not limit the technical scope or the significance of each term disclosed in the claims. Furthermore, dimensional ratios in the drawings are exaggerated for convenience of description, and may be different from actual ratios.

### <First Embodiment>

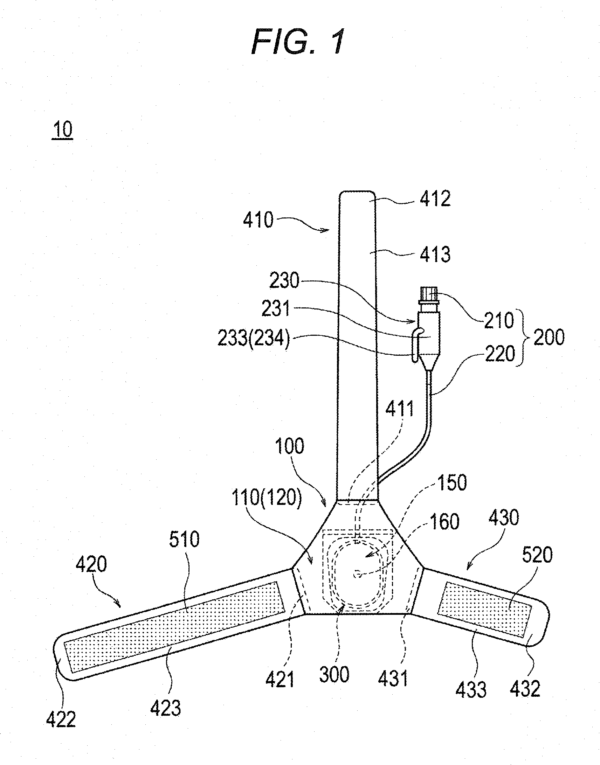

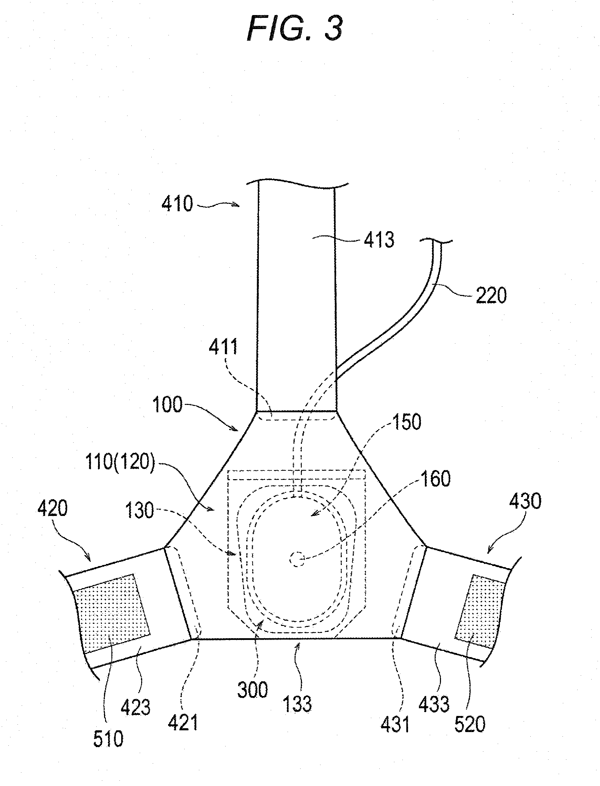

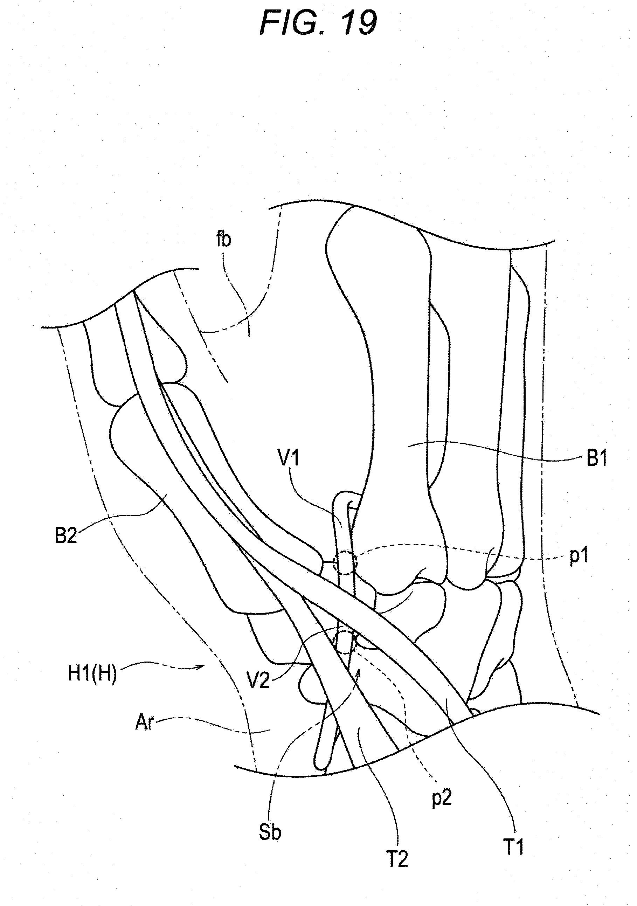

Figs. 1 to 18 are views for describing a hemostatic device 10 according to the present embodiment. Figs. 19 to 27 are views for describing a use example of the hemostatic device 10.

For example, as illustrated in Figs. 19, 22, 23, and 24, the hemostatic device 10 can be used to stop bleeding at a puncture site (hereinafter, also referred to as a "first puncture site p1") formed in a hand H located on a distal side (fingertip side) of a forearm Ar of a patient when a sheath tube 610 of an introducer 600 placed at the first puncture site p1 is removed.

A specific position of the puncture site where bleeding is to be stopped by the hemostatic device 10 is not particularly limited, but in the first embodiment, the first puncture site p1 and a second puncture site p2 are exemplified.

As illustrated in Figs. 19, 22, 23, and 24, the first puncture site p1 is a puncture site formed in a distal radial artery (hereinafter, also referred to as a "blood vessel V1") located on a distal side of a snuff box Sb of a palmar artery running on a dorsal side of a right hand H1 located on the distal side of the forearm Ar of the patient. Furthermore, the first puncture site p1 is located at a predetermined position avoiding a metacarpal bone B1 of an index finger and a metacarpal bone B2 of a thumb between the metacarpal bone B1 of the index finger and the metacarpal bone B2 of the thumb. Note that the snuff box Sb is a cavity of the hand located near a radius when the patient spreads the thumb of the hand H.

As illustrated in Fig. 19, the second puncture site p2 is a puncture site formed in an artery V2 located in the snuff box Sb of the palmar artery running on the dorsal side of the right hand H1. The second puncture site p2 is located on the forearm Ar side (proximal side) of the patient with respect to the first puncture site p1, and is located between an extensor pollicis longus muscle T1 and an extensor pollicis brevis muscle T2 located on the dorsal side of the right hand H1 of the patient. Furthermore, the second puncture site p2 is located at a predetermined position avoiding a position of the metacarpal bone B1 of the index finger and a position of the metacarpal bone B2 of the thumb.

Note that the hemostatic device 10 can also be applied to hemostasis of a puncture site on a left hand of a patient formed at a position corresponding to the first puncture site p1 exemplified with the patient's right hand H1 of the patient or a puncture site on the left hand of the patient formed at a position corresponding to the second puncture site p2 exemplified with the patient's right hand H1.

Hereinafter, the hemostatic device 10 will be described in detail.

### <Hemostatic Device>

As illustrated in Figs. 1, 2, 11, 22, 23, and 24, the hemostatic device 10 generally includes a cover member 100 configured to cover the first puncture site p1, an inflatable member 150 connected to the cover member 100 and configured to compress the first puncture site p1, an injection member 200 connected to the inflatable member 150 and configured to be capable of injecting a fluid into the inflatable member 150, and a securing member 230 configured to be capable of securing the injection member 200 to the cover member 100.

### <Inflatable Member>

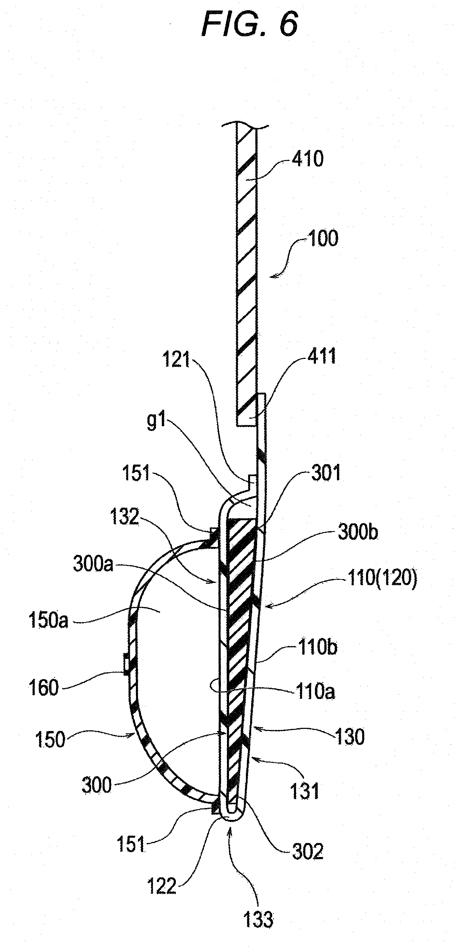

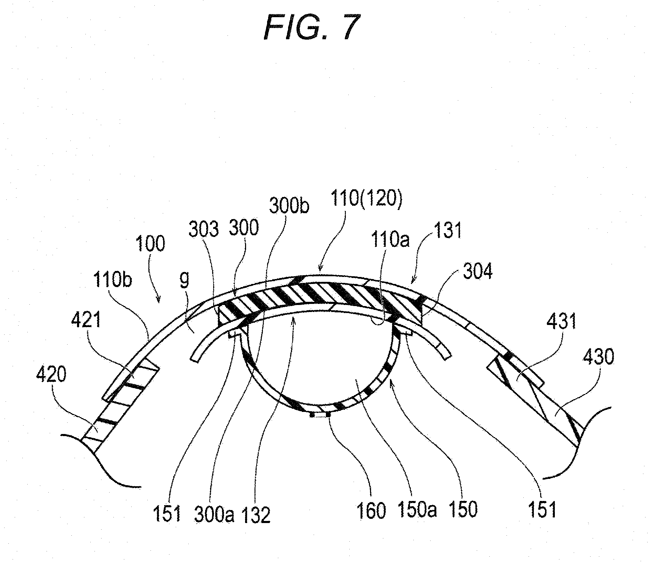

As illustrated in Figs. 6 and 7, the inflatable member 150 includes an inner cavity 150a into which the fluid can be injected. The inflatable member 150 is configured to inflate as the fluid is injected into the inner cavity 150a and deflate as the fluid injected into the inner cavity 150a is discharged. The fluid used for the inflation of the inflatable member 150 is, for example, a gas such as air. Figs. 6 and 7 illustrate cross-sectional views of the inflatable member 150 in an inflated state.

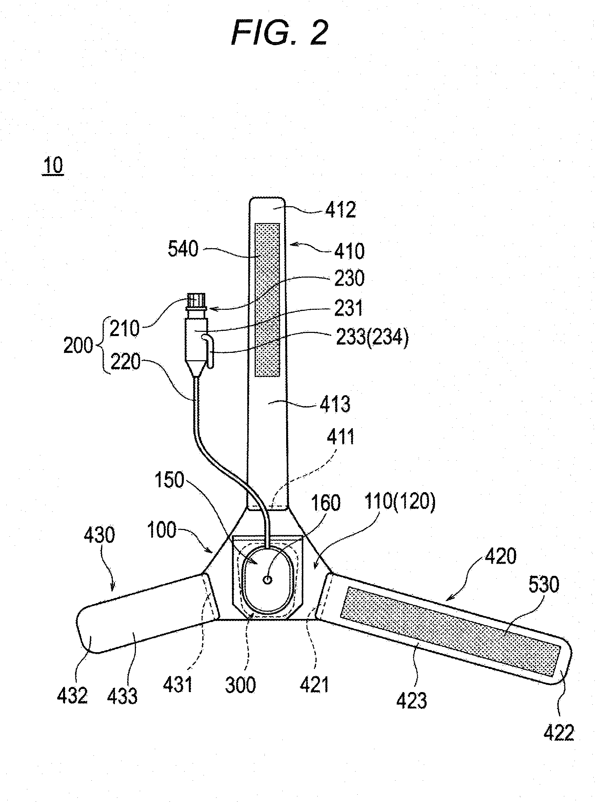

A tube member 220 (see Figs. 1 and 2) is coupled to the inner cavity 150a of the inflatable member 150.

As illustrated in Figs. 6 and 7, the inflatable member 150 can be formed of, for example, a film-shaped member (sheet material) connected to a sheet material 120 forming a main body 110 of the cover member 100.

The inflatable member 150 can be disposed to form the inner cavity 150a against a lower surface region 132 of a retaining portion 130 formed by the sheet material 120.

An outer peripheral edge of the film-shaped member forming the inflatable member 150 forms an edge portion 151 of the inflatable member 150. The edge portion 151 of the inflatable member 150 can be connected to the sheet material 120 by, for example, fusing bonding, adhesion, or the like.

The film-shaped member forming the inflatable member 150 can be made of, for example, a resin material having a predetermined thickness.

A material of the film-shaped member forming the inflatable member 150 is not particularly limited, and for example, polyvinyl chloride, polyethylene, polypropylene, polybutadiene, polyolefins such as ethylene-vinyl acetate copolymer (EVA), polyesters such as polyethylene terephthalate (PET) and polybutylene terephthalate (PBT), various thermoplastic elastomers such as polyvinylidene chloride, silicone, polyurethane, polyamide elastomer, polyurethane elastomer, polyester elastomer, nylon, nylon elastomer, or any combination thereof (such as a blend resin, a polymer alloy and a laminate) can be used. Note that the cover member 100 to be described later can be made of, for example, a material similar to each of those exemplified above.

As illustrated in Figs. 6 and 7, the inflatable member 150 is disposed on an inner surface 110a side of the main body 110 of the cover member 100.

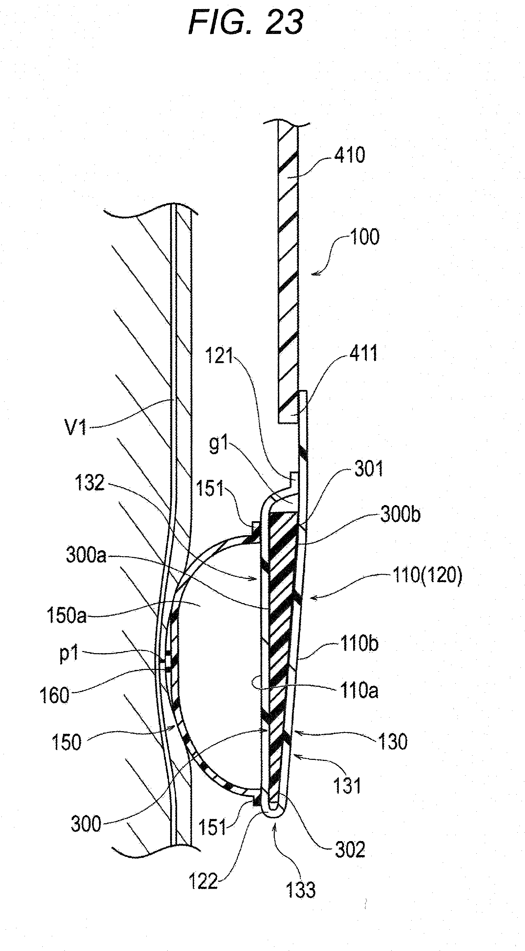

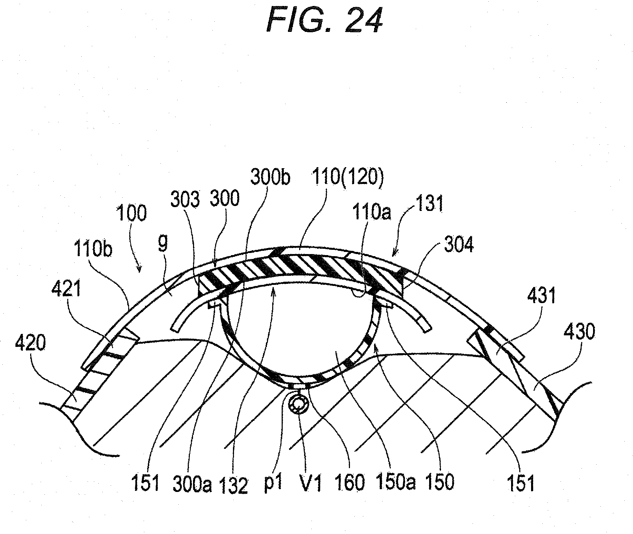

In the present embodiment, the inner surface 110a of the main body 110 is formed of a surface disposed on the body surface side of the right hand H1 in the lower surface region 132 of the retaining portion 130 (see Figs. 23 and 24). An outer surface 110b of the main body 110 is formed of a surface on a surface facing the outside in an upper surface region 131 of the retaining portion 130.

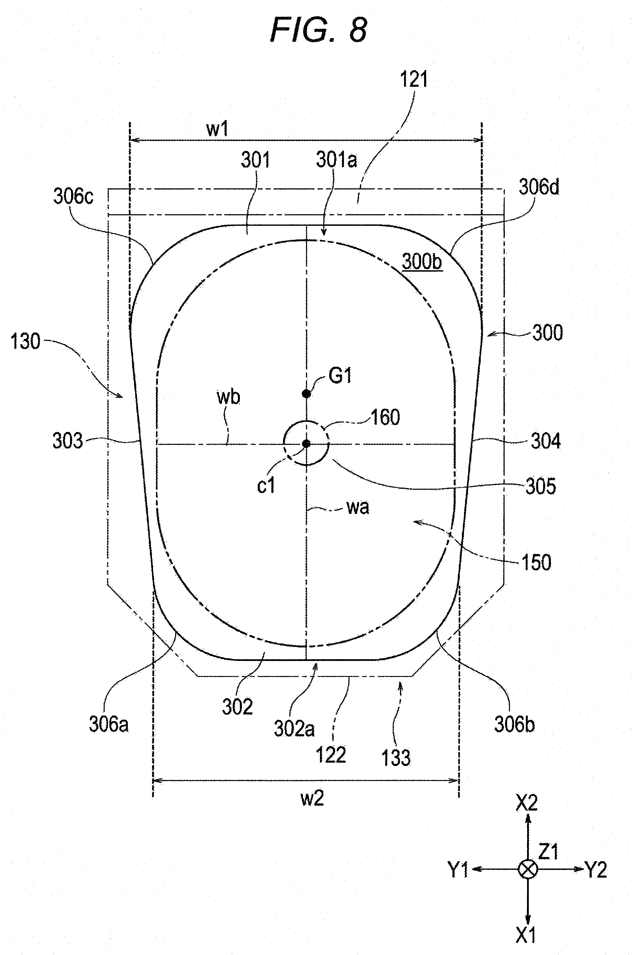

Fig. 8 is a plan view of a support member 300 as seen from an outer face 300b side. Fig. 8 illustrates a positional relationship among the inflatable member 150 in a deflated state, the support member 300, and the retaining portion 130. The inflatable member 150 and the retaining portion 130 are indicated by two-dot chain lines.

The inflatable member 150 has a longitudinal width wa passing through a center c1 of the inflatable member 150 and a lateral width wb that is orthogonal to the longitudinal width wa and passes through the center c1 of the inflatable member 150 in a state where the inflatable member 150 is deflated.

The center c1 of the inflatable member 150 is located at a center of an outer shape illustrated in the plan view of Fig. 8. For example, the inflatable member 150 can be formed such that the lateral width wb is shorter than the longitudinal width wa. In the present embodiment, the inflatable member 150 has a substantially oval shape that is rotationally symmetric with respect to the center c1 in the plan view illustrated in Fig. 8.

The inflatable member 150 can be disposed such that the center c1 of the inflatable member 150 is located closer to a lower end 302 of the support member 300 than a center of gravity G1 of the outer shape of the support member 300.

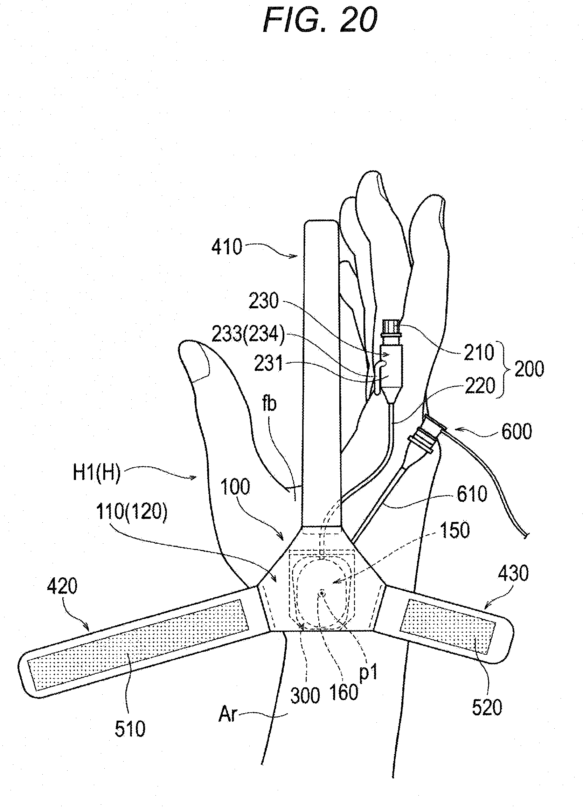

As illustrated in Figs. 20, 21, and 22, the inflatable member 150 includes a marker portion 160 for aligning the inflatable member 150 with the first puncture site p1.

In the present embodiment, the marker portion 160 is located at a position overlapping the center c1 of the inflatable member 150 as illustrated in Fig. 8.

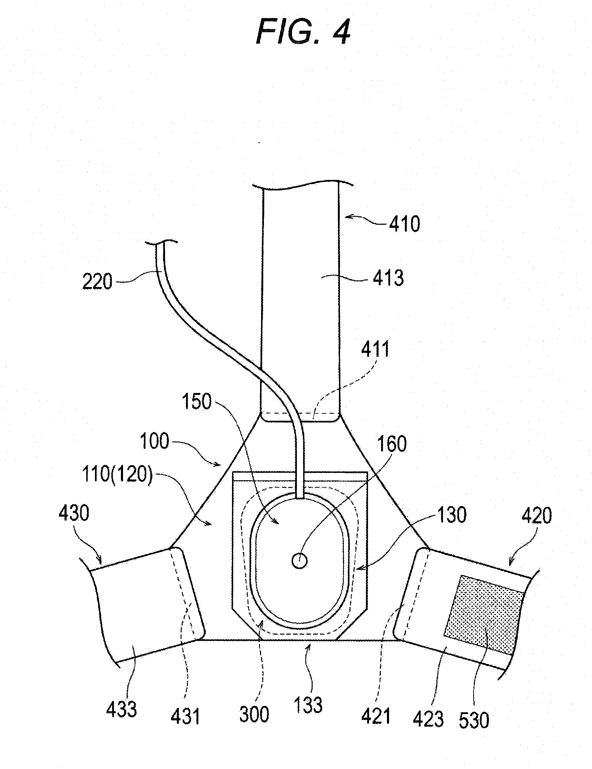

As illustrated in Figs. 4 and 8, the marker portion 160 can include a transparent central portion surrounding the center c1 and a colored circular frame portion surrounding the central portion.

As illustrated in Figs. 6 and 7, the marker portion 160 is disposed on an outer face of the inflatable member 150. Note that the marker portion 160 may be disposed on an inner face of the inflatable member 150 facing the inner cavity 150a.

A specific shape, color, formation method, or the like of the marker portion 160 is not particularly limited. The marker portion 160 can also include, for example, a circular marker configured entirely in color, a marker configured by a transparent central portion and a rectangular frame portion, a rectangular marker configured entirely in color, or the like.

The planar shape of the inflatable member 150 is not limited only to the oval shape. For example, the inflatable member 150 may have a planar shape such as a circle, an ellipse, or a rectangle.

A specific configuration of the inflatable member 150 is not particularly limited. For example, the inflatable member 150 may be formed by joining one sheet-like member with an edge portion of another sheet-like member in a state where the inner cavity 150a is formed between both the sheet-like members. In such a case, in the inflatable member 150, the surface of the one sheet-shaped member is connected to the sheet material 120 forming the main body 110 of the cover member 100. Furthermore, the inflatable member 150 can also be formed of, for example, one bag-shaped member shaped to include the inner cavity 150a inside. In such a case, in the inflatable member 150, a part of the surface of the bag-shaped member is connected to the sheet material 120 forming the main body 110 of the cover member 100.

### <Cover Member>

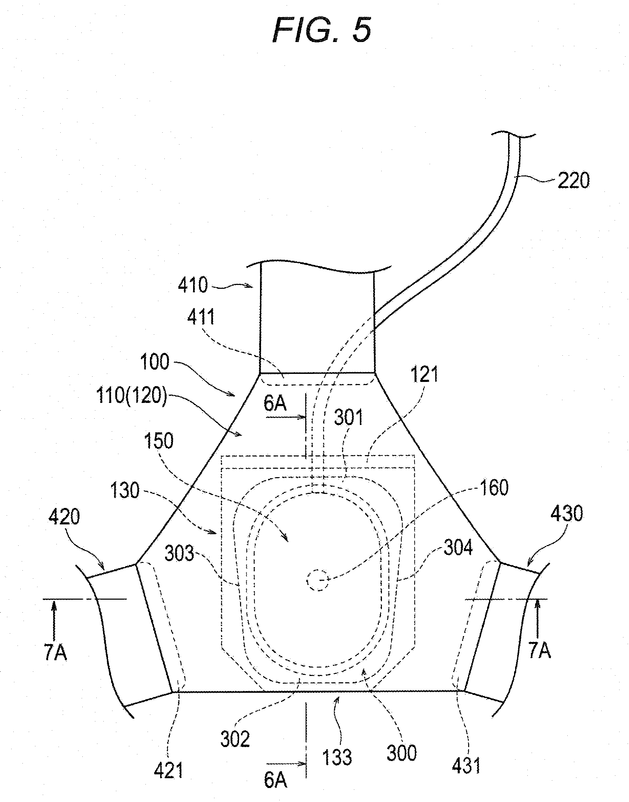

As illustrated in Figs. 5, 6, and 7, the cover member 100 includes the main body 110 in which the inflatable member 150 is located, a first band body 410 that is configured to be disposed between fingers of the patient and extends from the main body 110 in a first direction, a second band body 420 extending from the main body 110 in a second direction different from the first direction, and a third band body 430 facing the second band body 420 across the inflatable member 150 and extending from the main body 110 in a third direction different from the first direction and the second direction.

As illustrated in Figs. 5, 6, and 7, the main body 110 includes the support member 300 made of a material harder than the inflatable member 150. The support member 300 is configured to overlap the inflatable member 150 in the plan view illustrated in Figs. 4 and 5.

As illustrated in Figs. 6 and 7, the main body 110 has a two-layer structure and includes a retaining portion 130 retaining the support member 300.

The retaining portion 130 includes the upper surface region 131 located on the outer face 300b side of the support member 300, the lower surface region 132 that is located on an inner face 300a side of the support member 300 and faces the upper surface region 131 across the support member 300, and a curved region 133 connecting the upper surface region 131 and the lower surface region 132 on a proximal side of the support member 300 (the lower end 302 side of the support member 300).

The curved region 133 is formed by folding a part of the main body 110 toward the first band body 410 side. In the present embodiment, the retaining portion 130 having an insertion portion g into which the support member 300 can be inserted is configured by folding a part of the sheet material 120 forming the main body 110 from the proximal side to a distal side of the main body 110.

As illustrated in Figs. 5 and 6, a first end 121 located on the distal side of a folded part of the sheet material 120 is connected to the sheet material 120 at a position on the distal side of an upper end 301 of the support member 300.

As illustrated in Figs. 6 and 8, a proximal portion 122 of the cover member 100, formed of a part of the sheet material 120, is located in the curved region 133.

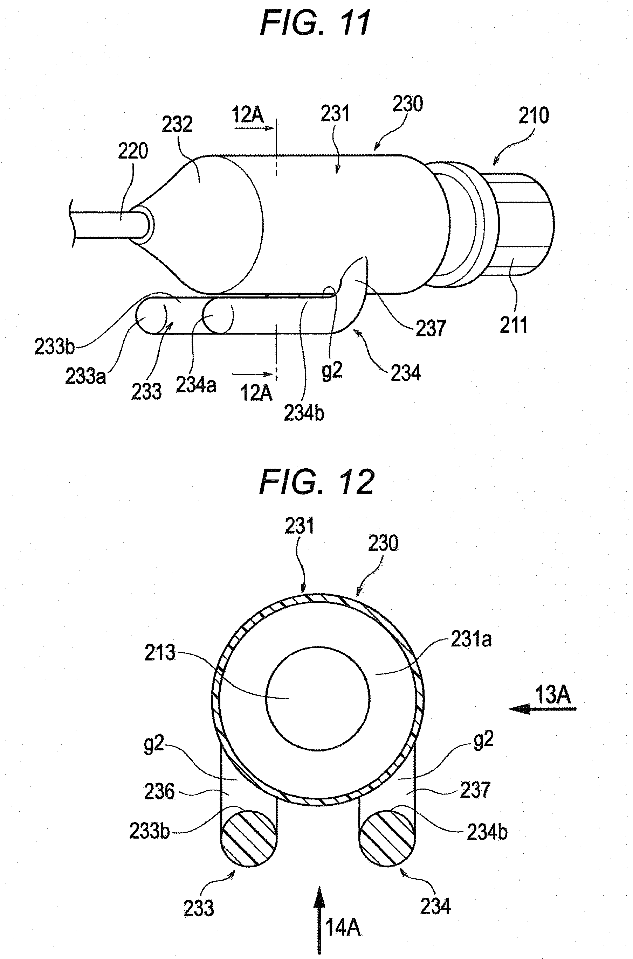

As illustrated in Fig. 6, in the retaining portion 130, the insertion portion g is closed between the first end 121 and the curved region 133 in a direction of the longitudinal width wa (see Fig. 11) of the inflatable member 150. Furthermore, as illustrated in Fig. 7, in the retaining portion 130, the insertion portion g communicates with the outside on the second band body 420 side and the third band body 430 side in a direction of the lateral width wb (see Fig. 11) of the inflatable member 150.

As illustrated in Fig. 4, each of the band bodies 410, 420, and 430 can be connected to any position in a region surrounding the periphery of the retaining portion 130 in the main body 110. Furthermore, each of the band bodies 410, 420, and 430 can be connected to a surface disposed on a body surface side of the right hand H1 in the above region. Each of the band bodies 410, 420, and 430 can be connected to the main body 110 by, for example, fusion bonding or adhesion. Note that each of the band bodies 410, 420, and 430 may be integrally formed with the main body 110.

As illustrated in Figs. 1, 2, 4, and 5, the first band body 410 includes one end 411 connected to the main body 110 of the cover member 100, the other end 412 located opposite to the one end 411, and a main body 413 extending between the one end 411 and the other end 412.

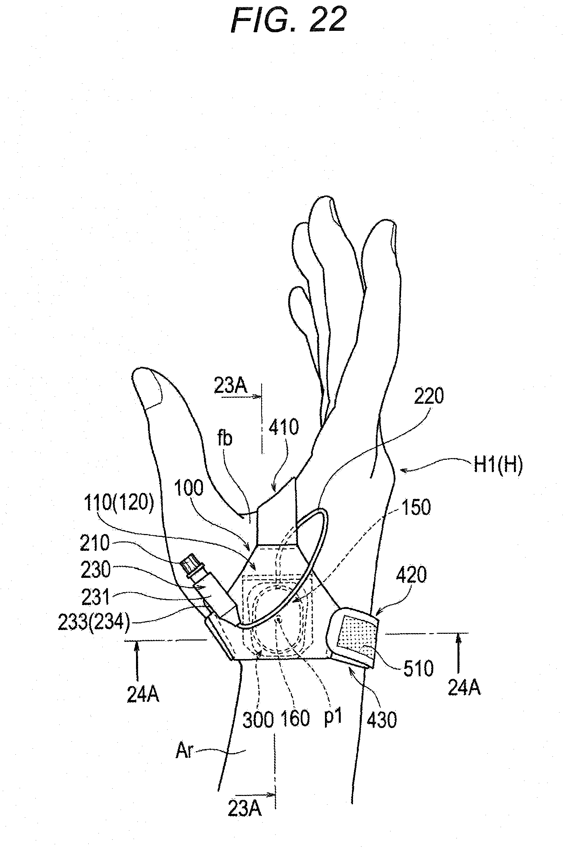

As illustrated in Fig. 22, the first band body 410 can be disposed so as to be hooked on an interdigital part fb located between the thumb and the index finger of the right hand H1 of the patient in a state where the inflatable member 150 is disposed at the first puncture site p1.

As illustrated in Figs. 1, 2, 4, and 5, the second band body 420 includes one end 421 connected to the main body 110 of the cover member 100, the other end 422 located opposite to the one end 421, and a main body 423 extending between the one end 421 and the other end 422.

As illustrated in Figs. 1, 2, 4, and 5, the third band body 430 includes one end 431 connected to the main body 110 of the cover member 100, the other end 432 located opposite to the one end 431, and a main body 433 extending between the one end 431 and the other end 432.

As illustrated in Fig. 22, the second band body 420 and the third band body 430 can be disposed so as to be wrapped along the outer periphery of the right hand H1 when the hemostatic device 10 is worn on the right hand H1 of the patient.

A constituent material of each of the band bodies 410, 420, and 430 is not particularly limited, and can be made of, for example, a vinyl chloride resin, a polyurethane resin, a polyester resin, or the like. Furthermore, a shape, a length, a thickness, and the like of each of the band bodies 410, 420, and 430 are not particularly limited.

Four securing parts, that is, a first securing part 510, a second securing part 520, a third securing part 530, and a fourth securing part 540, which enable the cover member 100 to be secured on the right hand H1, are disposed in the band bodies 410, 420, and 430, respectively.

As illustrated in Fig. 1, the first securing part 510 is disposed on an outer face of the main body 423 of the second band body 420.

As illustrated in Fig. 1, the second securing part 520 is disposed on an outer face of the main body 433 of the third band body 430.

As illustrated in Fig. 2, the third securing part 530 is disposed on an inner face of the main body 423 of the second band body 420.

As illustrated in Fig. 2, the fourth securing part 540 is disposed on an inner face of the main body 413 of the first band body 410.

The first securing part 510 and the second securing part 520 are formed of a male side of a hook-and-loop fastener. The third securing part 530 and the fourth securing part 540 are formed of a female side of the hook-and-loop fastener. The hook-and-loop fastener in the present specification is a hook-and-loop fastener, and is, for example, Magic Tape (registered trademark) or Velcro (registered trademark).

The second band body 420 and the third band body 430 are configured to be detachably attached via the third securing part 530 located on the inner face of the main body 423 of the second band body 420 and the second securing part 520 located on the outer face of the main body 433 of the third band body 430. Furthermore, the first band body 410 and the second band body 420 are configured to be detachably attached via the fourth securing part 540 located on the inner face of the main body 413 of the first band body 410 and the first securing part 510 located on the outer face of the main body 423 of the second band body 420.

Note that a specific structure of each of the securing parts 510, 520, 530, and 540 is not limited as long as the cover member 100 can be secured on the right hand H1 by connecting the band bodies 410, 420, and 430 to each other in a state where the hemostatic device 10 is disposed on the right hand H1. For example, omission of installation of some securing parts, a change of a position where the securing part is disposed in each of the band bodies 410, 420, and 430, and the like can be arbitrarily performed.

Furthermore, in a case where each of the securing parts 510, 520, 530, and 540 is formed of the hook-and-loop fastener, the male side and the female side of the hook-and-loop fastener may be interchanged. Furthermore, the securing parts 510, 520, 530, and 540 may be configured using, for example, coupling mechanisms or the like including a frame portion in which a snap, a button, a clip, or a protrusion is formed and an engaged portion in which a hole engageable with the frame portion is formed.

### <Support Member>

Figs. 8 to 10 illustrate the support member 300. In the drawings, arrows X1 and X2 indicate a longitudinal direction of the support member 300 (the same direction as the direction of the longitudinal width wa of the inflatable member 150), arrows Y1 and Y2 indicate a width direction of the support member 300 (the same direction as the direction of the lateral width wb of the inflatable member 150), and arrows Z1 and Z2 indicate a thickness direction of the support member 300.

As illustrated in Fig. 6, the support member 300 can be disposed in the insertion portion g of the retaining portion 130.

As illustrated in Figs. 4, 5, and 8, the support member 300 includes the upper end 301 that is located on the first band body 410 side and forms an end of the support member 300, the lower end 302 forming an end opposite to the upper end 301, and a pair of side surface portions 303 and 304 connecting the upper end 301 and the lower end 302.

As illustrated in Figs. 8 and 22, the upper end 301 can be disposed on a fingertip side (distal side) of the finger in a state where the hemostatic device 10 is worn on the right hand H1 of the patient. The lower end 302 can be disposed on the forearm Ar side (proximal side) in the state where the hemostatic device 10 is worn on the right hand H1 of the patient. Note that the upper end 301 forms a distal portion of the support member 300, and the lower end 302 forms a proximal portion of the support member 300.

The upper end 301 of the support member 300 has a flat portion 301a that is linear in the plan view illustrated in Fig. 8. The flat portion 301a extends substantially linearly between corners 306c and 306d located on the upper end 301 side.

The lower end 302 of the support member 300 has a flat portion 302a that is linear in the plan view illustrated in Fig. 8. The flat portion 302a extends substantially linearly between corners 306a and 306b located on the lower end 302 side.

The support member 300 has an intermediate portion 305 located between the upper end 301 and the lower end 302 in the plan view illustrated in Fig. 8. The intermediate portion 305 is located in the middle in the longitudinal direction connecting the upper end 301 and the lower end 302.

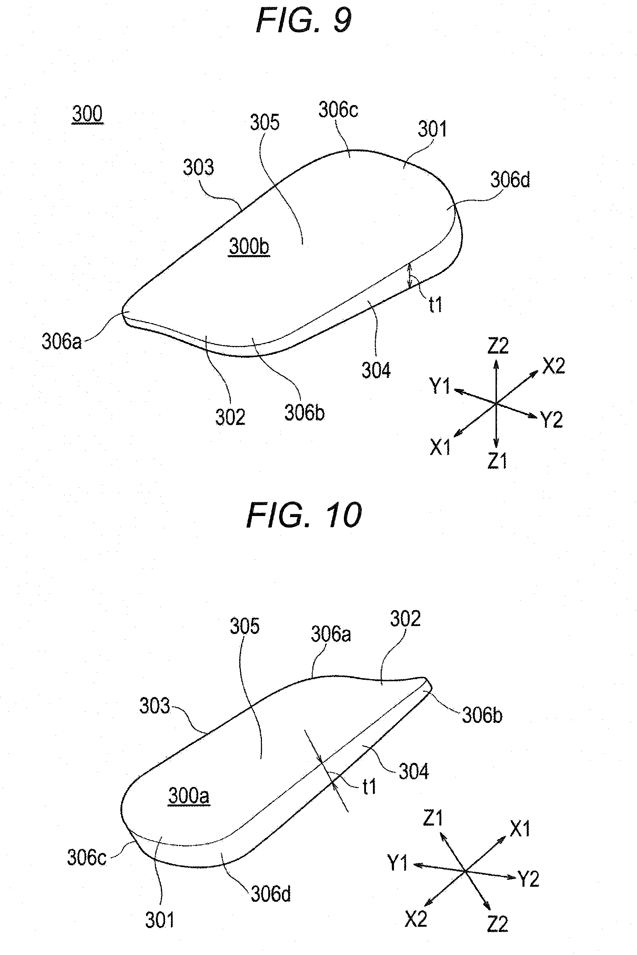

As illustrated in Figs. 7 and 9, the outer face 300b of the support member 300 is curved toward the second band body 420 and the third band body 430.

The outer face 300b of the support member 300 can be formed to have a substantially constant radius of curvature at each portion in the longitudinal direction of the support member 300, for example.

The support member 300 is located to have the center of gravity G1 of the outer shape of the support member 300 in the plan view illustrated in Fig. 8.

A width w1 of the upper end 301 is formed to be larger than a width w2 of the lower end 302 such that the center of gravity G1 is located on the upper end 301 side of the intermediate portion 305. In the present embodiment, the support member 300 has a substantially trapezoidal shape in which the width w1 of the upper end 301 is larger than the width w2 of the lower end 302.

Therefore, the center of gravity G1 of the support member 300 is located at a position shifted from the intermediate portion 305 of the support member 300 to the upper end 301 side (a lower bottom side of the trapezoidal shape) by a predetermined distance.

In this specification, as illustrated in Fig. 8, the width w1 of the upper end 301 of the support member 300 is defined by a dimension (maximum width) of a part having the largest width in a region located on the upper end 301 side of the intermediate portion 305 of the support member 300. Furthermore, the width w2 of the lower end 302 of the support member 300 can be defined by a length of a straight line connecting a boundary between the side surface portion 303 and the corner 306a and a boundary between the side surface portion 304 and the corner 306b. In a case where the corner 306a is formed in a curved shape as in the present embodiment, the boundary between the side surface portion 303 and the corner 306a is a position where a tangent line of the side surface portion 303 starts to change in a curved manner. Similarly, in a case where the corner 306b is formed in a curved shape as in the present embodiment, the boundary between the side surface portion 304 and the corner 306b is a position where a tangent line of the side surface portion 304 starts to change in a curved manner. Note that, in a case where the corners 306a and 306b are not formed in the curved shape, the width w2 of the lower end 302 of the support member 300 can be defined by a length of a straight line connecting apexes located at the corners 306a and 306b.

The inner face 300a of the support member 300 is curved toward the second band body 420 and the third band body 430. That is, as illustrated in Figs. 7 and 10, the inner face 300a of the support member 300 is curved in a shape projecting in a direction away from the inflatable member 150.

The inner face 300a of the upper end 301 of the support member 300 can be formed to have a radius of curvature smaller than a radius of curvature of the inner face 300a of the lower end 302 of the support member 300, for example.

As illustrated in Figs. 9 and 10, the support member 300 is configured such that a thickness t1 of each of the side surface portions 303 and 304 connecting the upper end 301 and the lower end 302 becomes thinner from the upper end 301 to the lower end 302. Therefore, the support member 300 is inclined such that a distance between the outer face 300b and the inner face 300a decreases from the upper end 301 to the lower end 302 in the cross-sectional view illustrated in Fig. 6 and a cross-sectional view illustrated in Fig. 23.

Each of the corners 306a, 306b, 306c, and 306d located at the four corners of the support member 300 is formed in a curved shape that is rounded.

The hemostatic device 10 is preferably formed such that a part where the marker portion 160 of the inflatable member 150 is provided, a part overlapping the marker portion 160 in the cover member 100 (the upper surface region 131 and the lower surface region 132 of the retaining portion 130), and a part overlapping the marker portion 160 in the support member 300 are transparent. In a case where each of the members 100, 150, and 300 is configured in this manner, the operator can easily visually confirm a position of the marker portion 160 disposed on the inflatable member 150 and/or the first puncture site p1 via the parts formed to be transparent in the members 100, 150, and 300 when the hemostatic device 10 is worn on the right hand H1 of the patient as illustrated in Figs. 20 to 22. Note that the term "transparent" in the present specification includes colored transparent, colorless transparent, and translucent.

As a constituent material of the support member 300 when the inflatable member 150 is made of the above-described material, for example, acrylic resin, polyvinyl chloride (particularly hard polyvinyl chloride), polyolefin such as polyethylene, polypropylene, or polybutadiene, polystyrene, poly- (4-methylpentene-1), polycarbonate, ABS resin, polymethyl methacrylate (PMMA), polyacetal, polyacrylate, polyacrylonitrile, polyvinylidene fluoride, ionomer, acrylonitrile-butadiene-styrene copolymer, polyethylene terephthalate (PET), or the like, can be used.

### <Injection Member>

As illustrated in Figs. 1, 2, and 11, the injection member 200 includes a connector portion 210 and the tube member 220 coupled to the connector portion 210 and the inflatable member 150.

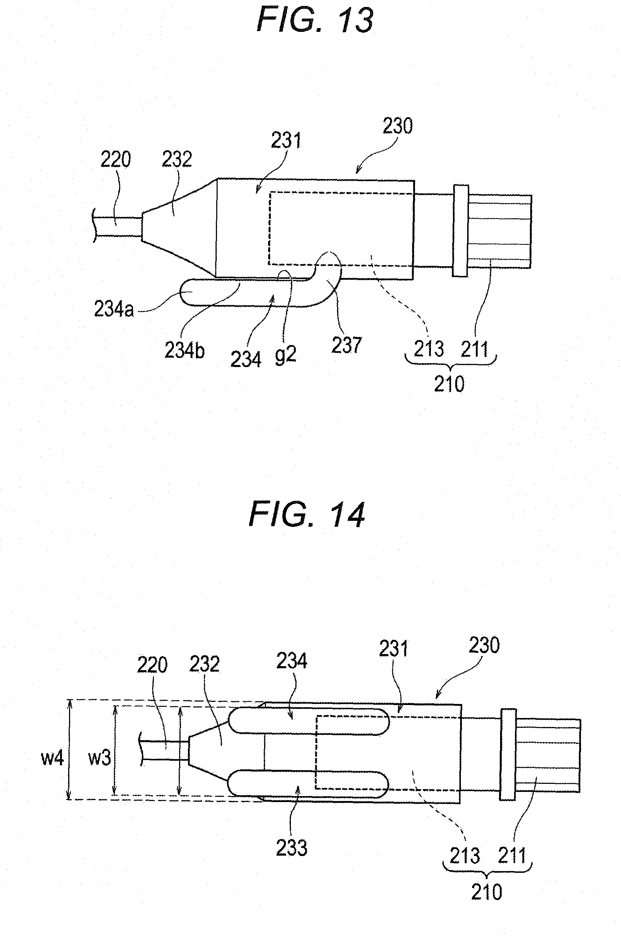

As illustrated in Fig. 13, the connector portion 210 includes a port portion 211 disposed outside the securing member 230 and a valve portion 213 disposed inside the main body 231 of the securing member 230. The valve portion 213 is briefly illustrated in each drawing, and is not illustrated in some drawings.

A fluid supply tool for supplying the fluid to the inner cavity 150a of the inflatable member 150 can be connected to the connector portion 210. As the fluid supply tool, for example, a syringe (not illustrated in the present specification) can be used.

The valve portion 213 can be formed of, for example, a check valve. The valve portion 213 maintains a closed state in a state where a cylindrical distal end portion of the syringe is not inserted into the connector portion 210. When the valve portion 213 is in the closed state, the valve portion 213 blocks communication between a lumen of the tube member 220 and the outside of the connector portion 210. The valve portion 213 is opened when the cylindrical distal end portion of the syringe is inserted into the connector portion 210. When the valve portion 213 is opened, the lumen of the tube member 220 communicates with the inside of the cylindrical distal end portion of the syringe via an internal space 231a (see Fig. 12) of the main body 231 in which the valve portion 213 is located.

As illustrated in Figs. 1 and 2, one end of the tube member 220 is coupled to the inflatable member 150. The other end of the tube member 220 is coupled to the main body 231 of the securing member 230. The other end of the tube member 220 is located in the internal space 231a of the main body 231 of the securing member 230.

The lumen of the tube member 220 communicates with the internal space 231a of the main body 231 via the other end of the tube member 220. Therefore, the inner cavity 150a of the inflatable member 150 communicates with the internal space 231a of the main body 231 of the securing member 230 via the lumen of the tube member 220.

When the inflatable member 150 is to be inflated, the operator inserts the cylindrical distal end portion of the syringe (not illustrated) into the connector portion 210 to open the valve portion 213. The operator injects air in the syringe into the inner cavity 150a of the inflatable member 150 by pushing a pusher of the syringe in a state where the valve portion 213 is open. When the air is injected into the inner cavity 150a, the inflatable member 150 inflates.

When the inflatable member 150 is to be deflated, the operator inserts the cylindrical distal end portion of the syringe into the connector portion 210 and further pulls the pusher of the syringe. The operator can discharge the air in the inner cavity 150a of the inflatable member 150 to the syringe by performing the above operation.

### <Securing Member>

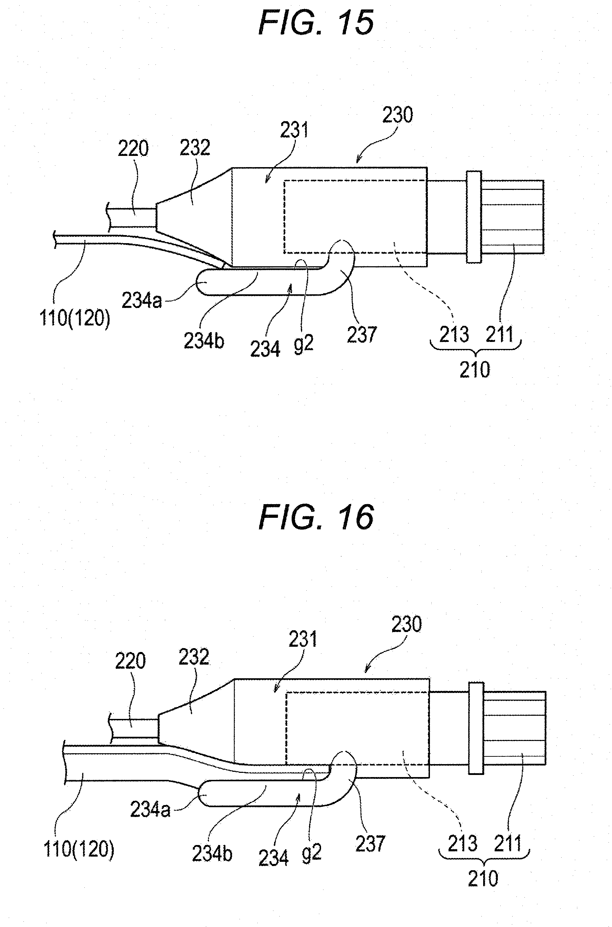

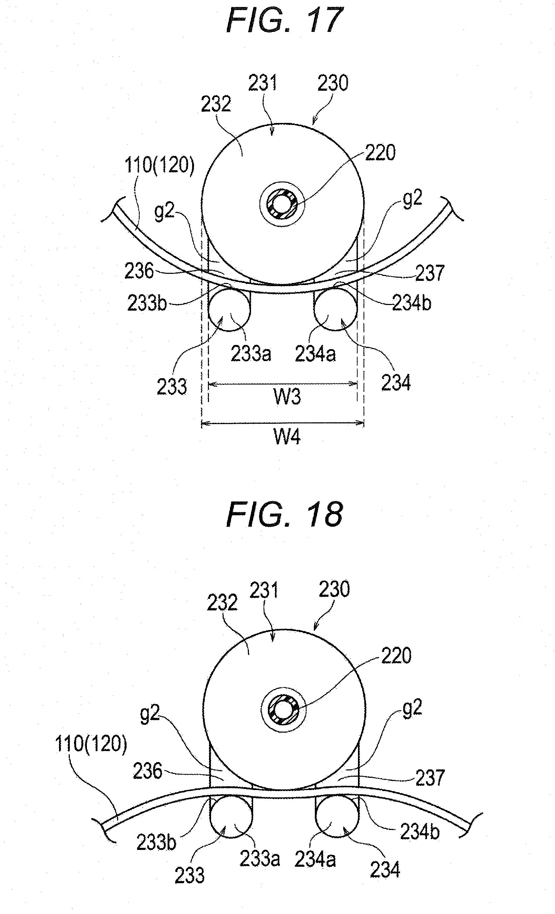

Figs. 11 to 14 illustrate the securing member 230 as seen from each direction. Figs. 15 and 16 illustrate states when the securing member 230 is secured to the cover member 100. Figs. 17 and 18 illustrate states where the securing member 230 is secured to the cover member 100.

As illustrated in Figs. 11, 14, and 16, the securing member 230 includes the main body 231 connecting the connector portion 210 and the tube member 220, claw portions 233 and 234 extending in parallel with the main body 231 toward the inflatable member 150 side, and coupling portions 236 and 237 that are configured to be capable of inserting the cover member 100 between the main body 231 and the claw portions 233 and 234, respectively, and couple the main body 231 and the claw portions 233 and 234, respectively.

The other end of the tube member 220 is coupled to the main body 231. Note that a side of the securing member 230 to which the other end of the tube member 220 is coupled is referred to as a "distal side", and a side of the securing member 230 on which the port portion 211 is disposed is referred to as a "proximal side".

As illustrated in Figs. 11 and 12, the main body 231 has the two claw portions 233 and 234. Hereinafter, the claw portion 233 is referred to as a "first claw portion 233", and the claw portion 234 is referred to as a "second claw portion 234".

The first claw portion 233 and the second claw portion 234 extend in parallel with each other. A gap g2 into which the cover member 100 can be inserted is formed between the first claw portion 233 and the main body 231 and between the second claw portion 234 and the main body 231.

As illustrated in Fig. 14, a width w3 of the first claw portion 233 and the second claw portion 234 is smaller than a width w4 of the main body 231 at positions where the first claw portion 233 and the second claw portion 234 overlap the main body 231 in a plan view. The above plan view means a plan view as seen from a bottom surface side illustrated in Fig. 14 or a plan view as seen from an upper surface side opposite to the bottom surface side.

In the present embodiment, the main body 231 has the two claw portions 233 and 234. When the main body 231 is configured as described above, the width w3 of the first claw portion 233 and the second claw portion 234 is defined by a distance between outermost peripheral positions of the claw portions 233 and 234 farthest from a position of a center of the main body 231 as illustrated in Figs. 14 and 17.

As illustrated in Fig. 17, the hemostatic device 10 can secure the securing member 230 to the cover member 100 by inserting the cover member 100 between the claw portions 233 and 234 and the gaps g2. When inserting the cover member 100 into the gaps g2, the operator can easily insert the cover member 100 into the gaps g2 by bringing the claw portions 233 and 234 close to the cover member 100 along a direction substantially parallel with a plane direction of the cover member 100. When the operator performs the above work, it is possible to prevent a force in a direction of pressing the cover member 100 against the body surface side of the right hand H1 of the patient from being inadvertently applied to the hemostatic device 10 when the securing member 230 is secured to the cover member 100.

Furthermore, since the width w3 of the first claw portion 233 and the second claw portion 234 of the securing member 230 is smaller than the width w4 of the main body 231, the following effects are obtained.

When the hemostatic device is worn on the right hand H1 of the patient, the cover member may be curved following a movement of the right hand H1 of the patient. For example, the cover member 100 is curved in a shape recessed toward the main body 231 side as illustrated in Fig. 17, or is curved in a shape projecting toward the main body 231 side as illustrated in Fig. 18. In a case where the cover member is curved as described above, if a radius of curvature of the cover member (a radius of curvature when a shape of the cover member in a front view is assumed to be an arc) is excessively small, the cover member easily interferes with the claw portions when the cover member is to be inserted into the gap g2. In particular, in a case where the width of the first claw portion and the second claw portion are larger than the width of the main body, if the cover member is curved in the shape projecting toward the main body side as illustrated in Fig. 18, the cover member more easily interferes with the first claw portion and the second claw portion. As a result, it is difficult to smoothly insert the cover member into the gap in the hemostatic device.

In the hemostatic device 10, the width w3 of the first claw portion 233 and the second claw portion 234 is smaller than the width w4 of the main body 231 at the positions where the first claw portion 233 and the second claw portion 234 overlap the main body 231 in the plan view. Therefore, even in the case where the cover member 100 is curved in the shape projecting toward the main body 231 side as described above, the hemostatic device 10 can suppress the interference of the first claw portion 233 and the second claw portion 234 with the cover member 100 when the cover member 100 is inserted into the gap g2.

As illustrated in Figs. 11 and 12, the main body 231 includes an inclined portion 232 inclined toward the claw portions 233 and 234.

The inclined portion 232 is configured such that at least a part of the inclined portion 232 is located on the proximal side of distal ends 233a and 234a of the claw portions 233 and 234. In the embodiment illustrated in Fig. 11, the inclined portion 232 extends from a position on the distal side of the distal ends 233a and 234a of the claw portions 233 and 234 to a position on the proximal side of the distal ends 233a and 234a of the claw portions 233 and 234. As illustrated in Figs. 15 and 16, when inserting the cover member 100 into the gap g2, the operator moves the cover member 100 toward the gap g2 from the position on the distal side of the distal ends 233a and 234a of the claw portions 233 and 234. At this time, the inclined portion 232 guides the cover member 100 toward the gap g2 along the inclined portion 232 while adjusting the shape of the cover member 100. Since the inclined portion 232 extends from the position on the distal side of the distal ends 233a and 234a of the claw portions 233 and 234 to the position on the proximal side of the distal ends 233a and 234a of the claw portions 233 and 234 in the embodiment of Fig. 11, the cover member 100 can be efficiently guided to positions of the distal ends 233a and 234a of the claw portions 233 and 234 along the inclined portion 232. The inclined portion 232 assists the insertion of the cover member 100 into the gap g2 by guiding the movement of the cover member 100. Therefore, the operator can smoothly insert the cover member 100 into the gap g2.

As illustrated in Fig. 11, the main body 231 can be configured to have a cylindrical outer shape. Furthermore, the main body 231 can be configured to have a circular cross-sectional shape in a direction orthogonal to an extending direction of the main body 231 as illustrated in Fig. 12.

The securing member 230 may receive various external forces in a state where the cover member 100 is inserted into the gap g2. The main body 231 of the securing member 230 has a circular cross-sectional shape. Therefore, when an external force is applied to the main body 231, the securing member 230 can divert the external force along an outer face of the main body 231. For example, in a case where the securing member 230 is formed to have a cross-sectional shape such as a quadrangle, when an external force is applied to the main body 231 as described above, the external force cannot be sufficiently diverted along the outer face of the main body 231. Therefore, there is a possibility that the hemostatic device 10 is displaced or inclined when the external force is applied to the main body 231.

Furthermore, since the main body 231 of the securing member 230 has the circular cross-sectional shape, it is possible to suitably prevent the main body 231 and the cover member 100 from interfering even in a case where the cover member 100 is formed in the curved shape recessed toward the main body 231 side as illustrated in Fig. 17 or a case where the cover member 100 is formed in the curved shape projecting toward the main body 231 side as illustrated in Fig. 18. For example, if the cover member 100 is curved as illustrated in Figs. 17 and 18 in a case where the main body 231 has a cross-sectional shape such as a quadrangle including corners, the cover member 100 interferes with the corners of the main body 231 when the cover member 100 is inserted into the gap g2 so that the cover member 100 cannot be smoothly inserted into the gap g2.

Note that the main body 231 can exhibit the above-described effect of diverting an external force even in a case where the main body has an elliptical cross-sectional shape. Therefore, the main body 231 is preferably formed to have the circular or elliptical cross-sectional shape from the viewpoint of exhibiting the effect of diverting an external force.

As illustrated in Figs. 11 and 12, each of the claw portions 233 and 234 has a cylindrical outer shape. The claw portions 223 and 234 have, on upper surfaces on the main body 231 side, flat portions 233b and 234b, respectively, configured to maintain a part of the cover member 100 in a flat shape in a state where the cover member 100 is inserted between the main body 231 and the claw portions 233 and 234.

Each of the flat portions 233b and 234b extends linearly in a side view illustrated in Fig. 13. Each of the flat portions 233b and 234b extends in substantially parallel with the extending direction of the main body 231 and faces the main body 231 across the gap g2 formed against the main body 231. When securing the securing member 230 to the cover member 100, the operator can easily insert and remove the cover member 100 into and from the gaps g2 along the flat portions 233b and 234b of the claw portions 233 and 234. Therefore, the operator can easily secure the securing member 230 to the cover member 100.

As illustrated in Figs. 11 and 13, the coupling portions 236 and 237 are curved from the main body 231 toward the distal ends 233a and 234a of the claw portions 233 and 234, respectively. Note that a shape of the coupling portion 236 is not clearly illustrated in Figs. 11 and 13, but the coupling portion 236 has substantially the same shape as the coupling portion 237, and is curved toward the distal end 233a side of the first claw portion 233 similarly to the coupling portion 237.

For example, the operator can cause the hemostatic device 10 to be worn on the patient in a state where the securing member 230 is secured to the cover member 100. Since each of the coupling portions 236 and 237 has the curved shape as described above, even if each of the coupling portions 236 and 237 comes into contact with the body surface or the like of the right hand H1 of the patient when the hemostatic device 10 is worn on the patient in a state where the securing member 230 is connected to the cover member 100, the hemostatic device 10 can prevent pain or the like from being given to the patient. Furthermore, since each of the coupling portions 236 and 237 has the curved shape as described above, the hemostatic device 10 can prevent each of the coupling portions 236 and 237 from biting into the skin when the patient performs an operation of touching a table stand with the right hand H1 or the like while the securing member 230 is secured to the cover member 100.

As illustrated in Fig. 11, the distal ends 233a and 234a of the claw portions 233 and 234 can be formed in a rounded shape. Since the distal ends 233a and 234a of the claw portions 233 and 234 are formed in the rounded shape in the securing member 230, it is possible to prevent the inflatable member 150 from being damaged in a case where the claw portions 233 and 234 unintentionally come into contact with the inflatable member 150 or the like. Furthermore, since the distal ends 233a and 234a of the claw portions 233 and 234 are formed in the rounded shape in the securing member 230, it is possible to reduce the pain felt by the patient even if the claw portions 233 and 234 erroneously hit the body surface of the patient when the securing member 230 is mounted on the cover member 100.

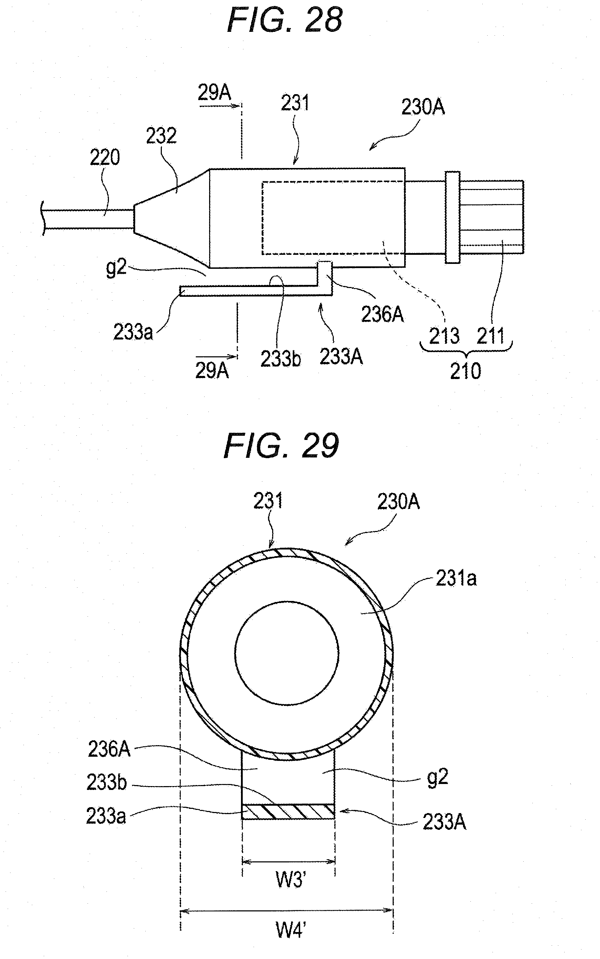

The securing member 230 according to the present embodiment has the two cylindrical claw portions 233 and 234. As will be described in a modification described later (see Figs. 28 and 29), the number and shapes of the claw portions 233 and 234 disposed on the securing member 230 are not particularly limited. However, in a case where a claw portion is formed in a cylindrical shape, it is preferable to form two or more claw portions 233 and 234 with a predetermined distance therebetween as illustrated in the present embodiment. This is for the following reason. For example, when the securing member 230 is configured to include one cylindrical claw portion, a contact area between the cover member 100 and the claw portion becomes excessively small in a state where the cover member 100 is inserted into the gap g2, and it becomes difficult to support the cover member 100 in a stable state between the claw portion and the main body 231. Even in the case where the claw portions are formed in the cylindrical shape as in the present embodiment, the cover member 100 can be supported in a stable state between each of the claw portions 233 and 234 and the main body 231 by providing the predetermined distance between the two claw portions 233 and 234.

Furthermore, a claw portion is preferably configured such that the cover member 100 inserted between the main body 231 and the claw portion can maintain a flat shape. When the cover member 100 inserted between the main body 231 and the claw can maintain the flat shape, the operability when the claw portion is inserted and removed with respect to the cover member 100 is improved. In order to achieve the above effect, claw portions are preferably disposed with a predetermined distance therebetween, for example, like the two claw portions 233 and 234 illustrated in the present embodiment. Since the predetermined distance is provided between the two claw portions 233 and 234, the securing member 230 can support the cover member 100 in a wider range in a direction in which the two claw portions 233 and 234 are arranged side by side.

As a result, the securing member 230 can maintain the cover member 100 in the flat shape between the main body 231 and each of the claw portions 233 and 234.

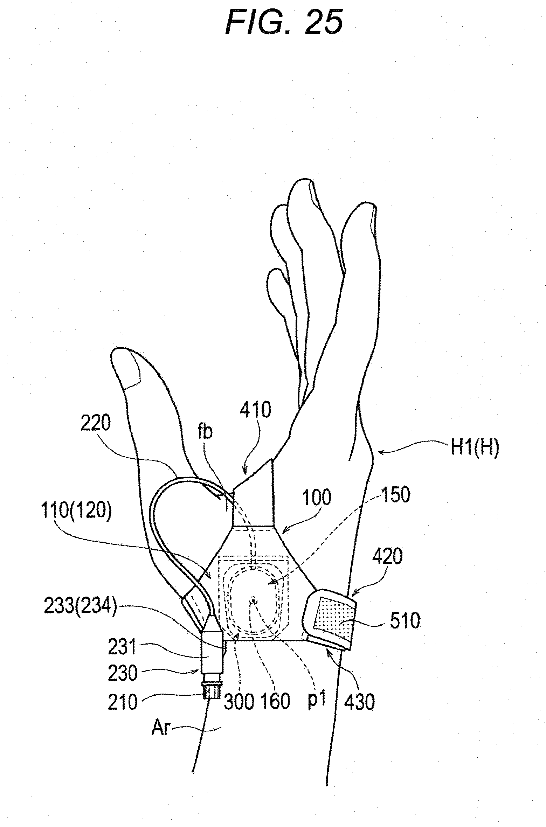

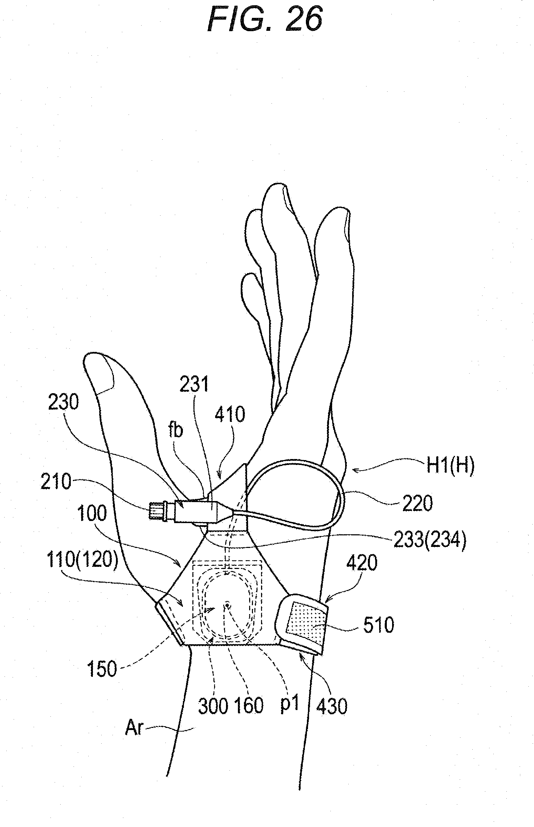

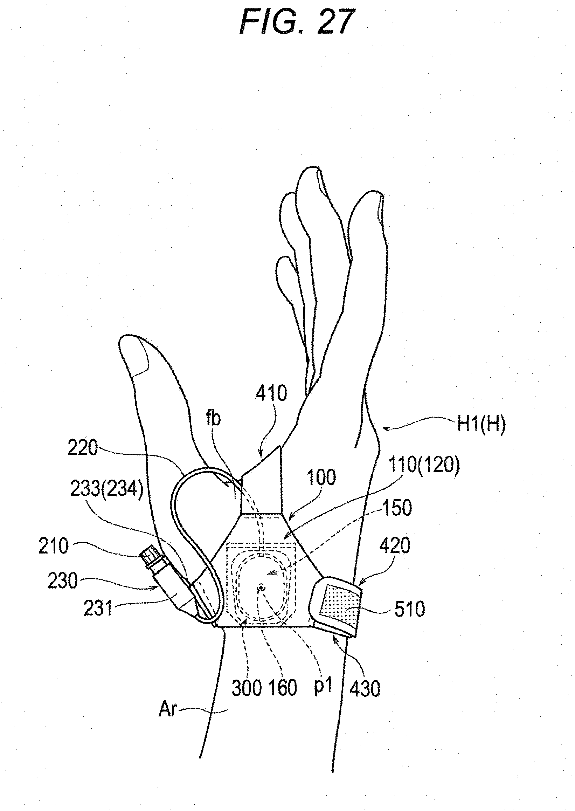

In performing hemostasis using the hemostatic device 10, a position at which the securing member 230 is secured to the cover member 100 is not particularly limited. For example, the securing member 230 can be secured at a position where a gap in which the claw portions 233 and 234 of the securing member 230 can be disposed is formed between the cover member 100 and the body surface of the right hand H1 when the inflatable member 150 inflates. As such a position, a part located between the first band body 410 and the second band body 420 in the main body 110 (see Fig. 22) or a part near the proximal portion 122 of the cover member 100 located on the second band body 420 side (see Fig. 25) can be selected. However, a position where the securing member 230 is connected to the cover member 100 is not particularly limited as long as the tube member 220 is not forcibly pulled when the inflatable member 150 inflates at the position, and for example, the securing member 230 may be secured to the first band body 410 as illustrated in Fig. 26, or the securing member 230 may be secured to the second band body 420 as illustrated in Fig. 27.

### <Use Example of Hemostatic Device>

Next, a use example of the hemostatic device 10 will be described with reference to Figs. 19 to 22.

Hereinafter, a process of using the hemostatic device 10 to stop bleeding at the first puncture site p1 formed on the right hand H1 of the patient illustrated in Fig. 19 will be described.

Fig. 20 illustrates a state where various procedures performed using the sheath tube 610 of the introducer 600 inserted into the first puncture site p1 are completed.

When causing the hemostatic device 10 to be worn on the right hand H1 of the patient, the operator disposes the inflatable member 150 and the support member 300 so as to overlap the first puncture site p1 as illustrated in Fig. 20. At this time, the operator can appropriately align the inflatable member 150 and the support member 300 with the first puncture site p1 by disposing the marker portion 160 at the first puncture site p1 while visually confirming a position of the marker portion 160 disposed at the inflatable member 150.

When causing the hemostatic device 10 to be worn on the right hand H1 of the patient, the operator can dispose the support member 300 so as to overlap the metacarpal bone B1 of the index finger and the metacarpal bone B2 of the thumb. An interval between the metacarpal bone B1 of the index finger and the metacarpal bone B2 of the thumb gradually narrows from the fingertip side to the forearm Ar side (see Fig. 19). A width of the support member 300 decreases from the upper end 301 disposed on the fingertip side to the lower end 302 disposed on the forearm Ar side (see Fig. 8). Therefore, the operator can dispose the support member 300 along the metacarpal bones B1 of the index finger and the metacarpal bones B2 of the thumb by disposing the support member 300 so as to overlap the metacarpal bones B1 of the index finger and the metacarpal bones B2 of the thumb from the dorsal side of the right hand H1 of the patient. The operator can prevent a gap from being formed between the inflatable member 150 disposed on the inner face 300a side of the support member 300 and the right hand H1 by disposing the support member 300 along the metacarpal bone B1 of the index finger and the metacarpal bone B2 of the thumb.

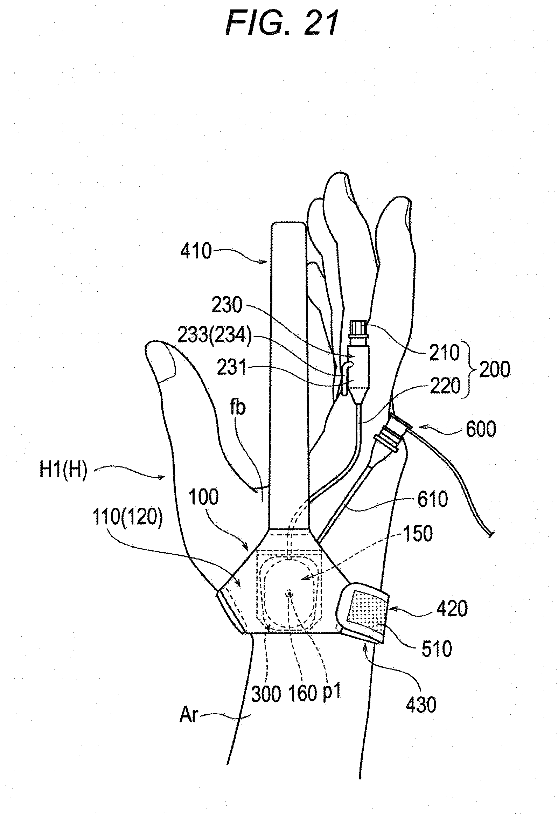

As illustrated in Fig. 21, the operator wraps the second band body 420 and the third band body 430 along the outer periphery of the right hand H1 of the patient. The operator can connect the second band body 420 and the third band body 430 via the securing parts 520 and 530 by bringing the third securing part 530 (see Fig. 2) disposed on the inner surface of the second band body 420 into contact with the second securing part 520 (see Fig. 1) disposed on the outer surface of the third band body 430. The operator can firmly wrap each of the band bodies 420 and 430 along the outer periphery of the right hand H1 by connecting the band bodies 420 and 430 to each other in a state where the support member 300 overlaps the metacarpal bone B1 of the index finger and the metacarpal bone B2 of the thumb.

The outer face 300b of the support member 300 is curved toward the second band body 420 side (the side surface portion 303 side) and the third band body 430 side (the side surface portion 304 side) (see Figs. 5, 7, and 9). Therefore, the operator can press the support member 300 against the right hand H1 from the outer face 300b side so as to be along the outer periphery of the right hand H1 as illustrated in Fig. 24 by wrapping the second band body 420 and the third band body 430 along the right hand H1 of the patient as illustrated in Fig. 21. Therefore, the hemostatic device 10 can enhance a force of securing the support member 300 and the inflatable member 150 with respect to the right hand H1 of the patient.

As illustrated in Fig. 22, the operator disposes a part of the first band body 410 on the palm side of the right hand H1 of the patient while passing the first band body 410 through the interdigital part fb located between the thumb and the index finger of the right hand H1 of the patient. At this time, the operator can connect the first band body 410 and the second band body 420 via the securing parts 510 and 540 by bringing the fourth securing part 540 (see Fig. 2) disposed on the inner face of the first band body 410 into contact with the first securing part 510 (see Fig. 1) disposed on the outer face of the second band body 420.

An inclined part exists in each portion (for example, the vicinity of the snuff box Sb) of the right hand H1 of the patient (see Fig. 19). Furthermore, a shape of the inclined part of the right hand H1 depends on an individual difference of the patient. For example, in a case where the inner face of the support member is formed in a flat shape, it is difficult to dispose the support member along the inclined part of the right hand H1. In the hemostatic device 10, the inner face 300a of the support member 300 is curved in the shape projecting in the direction away from the inflatable member 150 (see Figs. 7 and 24). Therefore, the inner face 300a of the support member 300 can be disposed along the right hand H1 of the patient by disposing the inner face 300a of the support member 300 so as to overlap the inclined part of the right hand H1 of the patient. As a result, the hemostatic device 10 can dispose the support member 300 to overlap the first puncture site p1 without depending on a dominant hand of the patient or the way of placement by the operator. Therefore, in the hemostatic device 10, the support member 300 can be appropriately disposed at a desired position near the first puncture site p1.

The operator connects the syringe to the port portion 211 in a state where the hemostatic device 10 is worn on the right hand H1 of the patient through the above process. The operator operates the syringe to inject air into the inflatable member 150, thereby inflating the inflatable member 150. As illustrated in Figs. 23 and 24, when the inflatable member 150 inflates in the hemostatic device 10, the inflatable member 150 applies a compressive force to the first puncture site p1.

The support member 300 made of the material harder than the inflatable member 150 presses the inflatable member 150 against the right hand H1 of the patient when the inflatable member 150 inflates in the state where the hemostatic device 10 is worn on the right hand H1 of the patient. As a result, the hemostatic device 10 can prevent the inflatable member 150 from lifting-off from the right hand H1 of the patient.

When performing hemostasis using the hemostatic device, the operator can strongly tighten the first band body disposed in the interdigital part fb, for example, in order to enhance the force of securing the support member with respect to the right hand H1 of the patient. The operator can firmly secure the support member to the right hand H1 of the patient by strongly tightening the first band body even if the support member is disposed in an unstable state on the right hand H1 of the patient. However, the patient sometimes feels pain when the first band body is strongly tightened. Therefore, in a case where the first band body cannot be strongly tightened due to the pain felt by the patient or the like, the hemostatic device may be disposed such that the inner face of the support member is inclined with respect to the body surface of the right hand H1 of the patient. Specifically, the upper end of the support member on which the first band body is disposed lifts-off to a position excessively away from the body surface of the right hand H1 of the patient as compared with the lower end located opposite to the upper end.

As a result, in the hemostatic device, the support member is disposed to be inclined such that a distance between the inner face of the support member and the body surface of the right hand H1 of the patient gradually decreases from the upper end to the lower end of the support member.

The hemostatic device 10 is configured such that the thickness t1 of each of the side surface portions 303 and 304 of the support member 300 becomes thinner from the upper end 301 to the lower end 302. In addition, the first band body 410 configured to be disposed at the interdigital part fb located between the fingers of the patient extends from the upper end 301 side of the support member 300, and is configured to press the upper end 301 side of the support member 300 against the right hand H1 of the patient. In the hemostatic device 10 configured as described above, when the inflatable member 150 starts to inflate in a state where the support member 300 and the inflatable member 150 are secured to the right hand H1 of the patient, the inflatable member 150 applies a force to the vicinity of the upper end 301 of the support member 300. The upper end 301 side of the support member 300 is lifted by the force applied by the inflatable member 150. As a result, the support member 300 is disposed such that the inner face 300a of the support member 300 is substantially parallel with the body surface of the right hand H1 of the patient in a state where the inflatable member 150 inflates as illustrated in Fig. 23. Therefore, the hemostatic device 10 can apply a compressive force in a direction perpendicular to the first puncture site p1 when the inflatable member 150 inflates.

The inner face 300a of the support member 300 is curved toward the second band body 420 side and the third band body 430 side (see Fig. 7). As illustrated in Fig. 24, the hemostatic device 10 is disposed such that the inner face 300a of the support member 300 is along the body surface of the right hand H1 of the patient in a state where the inflatable member 150 inflates. When the inflatable member 150 inflates, the hemostatic device 10 presses the inflatable member 150 along the body surface of the right hand H1 of the patient by the inner face 300a of the support member 300. As a result, when the inflatable member 150 inflates, the inflatable member 150 applies a compressive force in a direction toward the first puncture site p1 from a center side in the width direction of the inner face 300a of the support member 300 and the side surface portions 303 and 304 located at both ends in the width direction of the inner face 300a of the support member 300. Therefore, the hemostatic device 10 can effectively enhance the compressive force applied to the first puncture site p1 by the inflatable member 150 as compared with a case where the inner face 300a of the support member 300 is formed in a flat surface shape.

After the inflatable member 150 inflates, the operator removes the sheath tube 610 of the introducer 600 from the first puncture site p1 as illustrated in Fig. 22. The operator confirms that there is no bleeding from the first puncture site p1 during hemostasis performed using the hemostatic device 10. In a case where there is bleeding from the first puncture site p1, the operator can adjust an injection amount of air into the inflatable member 150.

Through the above process, the operator can stop bleeding at the first puncture site p1 formed on the right hand H1 of the patient using the hemostatic device 10.

The operator can secure the securing member 230 to the cover member 100 as illustrated in Fig. 22 in a state where the inflatable member 150 inflates. The operator can prevent the tube member 220 from moving following the movement of the right hand H1 of the patient in the state where the hemostatic device 10 is worn by securing the securing member 230 to the cover member 100. Note that the operator can also secure the securing member 230 to the cover member 100 in advance before inflating the inflatable member 150, and inflate the inflatable member 150 after the hemostatic device 10 is worn on the right hand H1 of the patient.

As described above, the hemostatic device 10 according to the present embodiment includes the cover member 100 configured to cover the first puncture site p1, the inflatable member 150 connected to the cover member 100 and configured to compress the first puncture site p1, the injection member 200 connected to the inflatable member 150 and configured to be capable of injecting a fluid into the inflatable member 150, and the securing member 230 configured to be capable of securing the injection member 200 to the cover member 100. The injection member 200 includes the connector portion 210 and the tube member 220 coupled to the inflatable member 150. The securing member 230 includes the main body 231 connecting the connector portion 210 and the tube member 220, the claw portions 233 and 234 extending in parallel with the main body 231 toward the inflatable member 150 side, and the coupling portions 236 and 237 that are configured to be capable of inserting the cover member 100 between the main body 231 and the claw portions 233 and 234, respectively, and couple the main body 231 and the claw portions 233 and 234, respectively. The width w3 of the claw portions 233 and 234 is smaller than the width w4 of the main body 231 at the positions where the claw portions 233 and 234 overlap the main body 231 in the plan view.

In the hemostatic device 10 configured as described above, the injection member 200 can be secured to the cover member 100 by inserting the cover member 100 between the main body 231 and the claw portions 233 and 234 in the state where the main body 231 of the securing member 230 connects the connector portion 210 and the tube member 220. When inserting the cover member 100 between the main body 231 and the claw portions 233 and 234, the operator can insert the cover member 100 between the main body 231 and the claw portions 233 and 234 by bringing the securing member 230 close to the cover member 100 along the direction substantially parallel with the plane direction of the cover member 100. Therefore, when securing the injection member 200 to the cover member 100, the operator can prevent an unnecessary force from being applied in a direction of pressing the hemostatic device 10 against the body surface of the right hand H1 of the patient. Therefore, when the injection member 200 is secured to the cover member 100, the hemostatic device 10 can prevent the hemostatic device 10 worn on the right hand H1 of the patient from being displaced or the inflatable member 150 from being displaced. Furthermore, for example, the cover member 100 may be curved following the movement of the body in a state where the hemostatic device 10 is worn on the patient. If the cover member 100 is curved in a case where the width w3 of the claw portions 233 and 234 is formed to be larger than the width w4 of the main body 231 at the positions where the claw portions 233 and 234 overlap the main body 231, the claw portions 233 and 234 easily interfere with the cover member 100 when the cover member 100 is inserted between the main body 231 and the claw portions 233 and 234. In the hemostatic device 10, the width w3 of the claw portions 233 and 234 is smaller than the width w4 of the main body 231 at the positions where the claw portions 233 and 234 overlap the main body 231 in the plan view. Therefore, in the hemostatic device 10 described above, the cover member 100 can be smoothly inserted between the main body 231 and the claw portions 233 and 234 even when the cover member 100 is curved following the movement of the right hand H1 of the patient.

Furthermore, the hemostatic device 10 according to the present embodiment includes the cover member 100 configured to cover the first puncture site p1, the inflatable member 150 connected to the cover member 100 and configured to compress the first puncture site p1, the injection member 200 connected to the inflatable member 150 and configured to be capable of injecting a fluid into the inflatable member 150, and the securing member 230 configured to be capable of securing the injection member 200 to the cover member 100. The injection member 200 includes the connector portion 210 and the tube member 220 coupled to the inflatable member 150. The securing member 230 includes the main body 231 connecting the connector portion 210 and the tube member 220, the claw portions 233 and 234 extending in parallel with the main body 231 toward the inflatable member 150 side, and the coupling portions 236 and 237 that are configured to be capable of inserting the cover member 100 between the main body 231 and the claw portions 233 and 234, respectively, and couple the main body 231 and the claw portions 233 and 234, respectively. The main body 231 has the inclined portion 232 inclined toward the claw portions 233 and 234, and at least a part of the inclined portion 232 is configured to be located at the position on the proximal side of the distal ends 233a and 234a of the claw portions 233 and 234 in the main body 231.

In the hemostatic device 10 configured as described above, the injection member 200 can be secured to the cover member 100 by inserting the cover member 100 between the main body 231 and the claw portions 233 and 234 in the state where the main body 231 of the securing member 230 connects the connector portion 210 and the tube member 220. When inserting the cover member 100 between the main body 231 and the claw portions 233 and 234, the operator can insert the cover member 100 between the main body 231 and the claw portions 233 and 234 by bringing the securing member 230 close to the cover member 100 along the direction substantially parallel with the plane direction of the cover member 100. Therefore, when securing the injection member 200 to the cover member 100, the operator can prevent an unnecessary force from being applied in a direction of pressing the hemostatic device 10 against the body surface of the right hand H1 of the patient.

Therefore, when the injection member 200 is secured to the cover member 100, the hemostatic device 10 can prevent the hemostatic device 10 worn on the right hand H1 of the patient from being displaced or the inflatable member 150 from being displaced. Furthermore, the hemostatic device 10 is configured such that the main body 231 has the inclined portion 232 inclined toward the claw portions 233 and 234. Furthermore, at least a part of the inclined portion 232 of the main body 231 is configured to be located at the position on the proximal side of the distal ends 233a and 234a of the claw portions 233 and 234 in the main body 231. Therefore, the hemostatic device 10 is configured such that when the operator inserts the cover member 100 between the main body 231 and the claw portions 233 and 234, the inclined portion 232 guides the cover member 100 to a position closer to the coupling portions 236 and 237 than the distal ends 233a and 234a of the claw portions 233 and 234. Therefore, when inserting the cover member 100 between the main body 231 and the claw portions 233 and 234, the operator can smoothly insert the cover member 100 between the main body 231 and the claw portions 233 and 234 while adjusting the shape of the cover member 100 by guiding the cover member 100 along the inclined portion 232.

The main body 231 has the inclined portion 232 inclined toward the claw portions 233 and 234. The inclined portion 232 extends from the position on the distal side of the distal ends 233a and 234a of the claw portions 233 and 234 to the position on the proximal side of the distal ends 233a and 234a of the claw portions 233 and 234.

According to the hemostatic device 10 configured as described above, the inclined portion 232 efficiently guides the cover member 100 to the positions of the distal ends 233a and 234a of the claw portions 233 and 234 when the cover member 100 is inserted into the gap g2. The inclined portion 232 assists the insertion of the cover member 100 into the gap g2 when the cover member 100 is inserted into the gap g2. Therefore, the operator can smoothly insert the cover member 100 into the gap g2.

The main body 231 can be configured to have a circular or elliptical cross-sectional shape.

According to the hemostatic device 10 configured as described above, when an external force is applied to the main body 231, the securing member 230 can divert the external force along the outer face of the main body 231. Therefore, it is possible to prevent the hemostatic device 10 from being displaced or inclined when the external force is applied to the main body 231. Furthermore, even in a case where the cover member 100 is formed in the curved shape recessed toward the main body 231 side or the cover member 100 is formed in the curved shape projecting toward the main body 231 side, it is possible to suitably prevent the main body 231 and the cover member 100 from interfering when the cover member 100 is inserted into the gap g2.

The claw portions 233 and 234 have the flat portions 233b and 234b, respectively, configured to maintain a part of the cover member 100 in a flat shape in a state where the cover member 100 is inserted between the main body 231 and the claw portions 233 and 234. Each of the flat portions 233b and 234b faces the main body 231 across the gap g2 formed between the main body 231 and each of the claw portions 233 and 234.

According to the hemostatic device 10 configured as described above, when securing the securing member 230 to the cover member 100, the operator can easily insert and remove the cover member 100 into and from the gaps g2 along the flat portions 233b and 234b of the claw portions 233 and 234. Therefore, the operator can easily secure the securing member 230 to the cover member 100.

Each of the coupling portions 236 and 237 has a shape curved from the main body 231 toward each of the distal ends 233a and 234a of the claw portions 233 and 234.

According to the hemostatic device 10 configured as described above, even if each of the coupling portions 236 and 237 comes into contact with the body surface or the like of the right hand H1 of the patient when the hemostatic device 10 is worn on the patient in a state where the securing member 230 is connected to the cover member 100, the hemostatic device 10 can prevent pain or the like from being given to the patient.

Furthermore, since each of the coupling portions 236 and 237 has the curved shape as described above, the hemostatic device 10 can prevent each of the coupling portions 236 and 237 from biting into the skin when the patient performs an operation of touching a table stand with the right hand H1 or the like while the securing member 230 is secured to the cover member 100.

The cover member 100 includes the main body 110 in which the inflatable member 150 is located, the first band body 410 that is configured to be disposed between fingers of the patient and extends from the main body 110 in the first direction, the second band body 420 extending from the main body 110 in the second direction different from the first direction, and the third band body 430 facing the second band body 420 across the inflatable member 150 and extending from the main body 110 in a different direction from the first band body 410 and the second band body 420. The claw portions 233 and 234 are configured such that any of the main body 110 of the cover member 100, the first band body 410, the second band body 420, and the third band body 430 can be inserted between the claw portions and the coupling portions 236 and 237.

According to the hemostatic device 10 configured as described above, the securing member 230 can be secured to any position of the cover member 100 provided in the hemostatic device 10 configured to be capable of being worn on the right hand H1. Therefore, the convenience of the hemostatic device 10 is improved.

Next, modifications of the above-described first embodiment will be described. In the description of the modifications, the description regarding the members and the process of using the hemostatic device already described in the first embodiment will be appropriately omitted. Furthermore, contents that are not particularly described in the modifications can be the same as those in the first embodiment.

### <First Modification>

Fig. 28 is a side view of a securing member 230A according to a first modification. Fig. 29 is a cross-sectional view taken along an arrow 29A-29A illustrated in Fig. 28.

As illustrated in Figs. 28 and 29, the securing member 230A includes a claw portion 233A.

The claw portion 233A includes a flat portion 233b configured to maintain a part of the cover member 100 in a flat shape in a state where the cover member 100 is inserted between the main body 231 and the claw portion 233A. As illustrated in Fig. 29, in the claw portion 233A of the present modification, the flat portion 233b extends in parallel with an extending direction of the main body 231. Furthermore, the flat portion 233b extends linearly in the side view illustrated in Fig. 28. The flat portion 233b faces the main body 231 across the gap g2 formed between the main body 231 and the claw portion 233A.

The securing member 230A includes the single claw portion 233A. The claw portion 233A is coupled to the main body 231 via a coupling portion 236A.

As illustrated in Fig. 29, the claw portion 233A has a rectangular shape in a front view. The flat portion 233b forms an upper surface of the claw portion 233A located on the main body 231 side. Furthermore, as illustrated in the cross-sectional view of Fig. 29, the flat portion 233b of the first modification forms a plane extending linearly in a width direction of the claw portion 233A. Therefore, the claw portion 233A can maintain a part of the cover member 100 in the flat shape in a relatively wide range in the state where the cover member 100 is inserted between the main body 231 and the claw portion 233A. The claw portion 233A extends substantially linearly toward the distal end 233a. The coupling portion 236A can be configured to extend substantially linearly between the main body 231 and the claw portion 233A, for example.

As illustrated in Fig. 29, a width w3' of the claw portion 233A is smaller than a width w4' of the main body 231 at a position where the claw portion 233A overlaps the main body 231 in the front view. Note that the above-described magnitude relationship of the widths in the front view is similar also in a plan view of the securing member 230A.

The claw portion 233A of the securing member 230A according to the first modification includes the linear flat portion 233b facing the main body 231 across the gap g2 formed between the main body 231 and the claw portion 233A. The claw portion 233A of the securing member 230A has a simple structure extending linearly. When connecting the securing member 230A to the cover member 100, an operator can easily insert and remove the cover member 100 into and from the gap g2 along the flat portion 233b. Therefore, the operator can easily secure the securing member 230A to the cover member 100.

### <Second Modification>

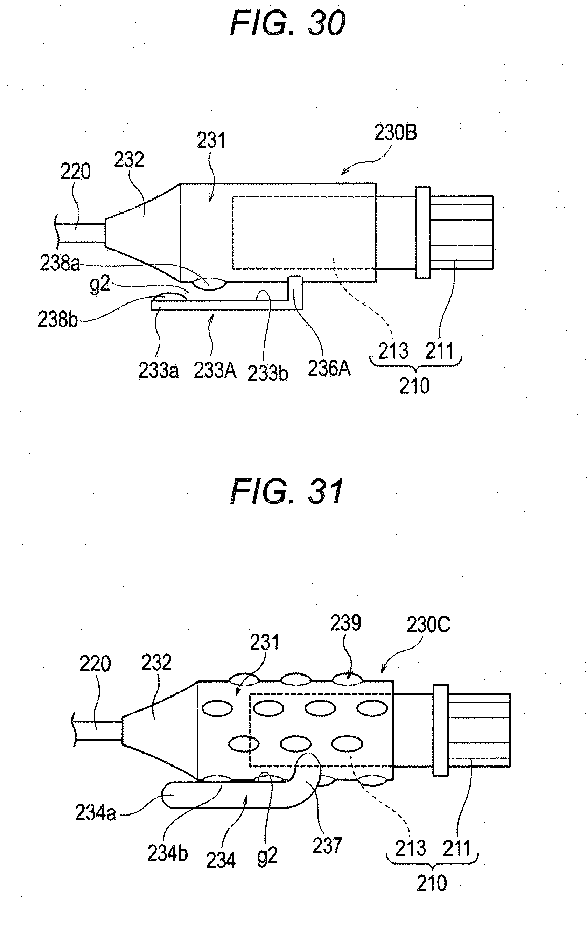

Fig. 30 is a side view of a securing member 230B according to a second modification.

The securing member 230B according to the second modification includes a first projection 238a formed on the main body 231 and a second projection 238b formed on the claw portion 233A. The first projection 238a protrudes from the main body 231 toward the gap g2. The second projection 238b protrudes from the claw portion 233A toward the gap g2.

The first projection 238a can be disposed, for example, at a position on the proximal side of the securing member 230B with respect to the second projection 238b. Furthermore, the second projection 238b can be disposed at the distal end 233a of the claw portion 233A.

In the securing member 230B according to the second modification, the projections 238a and 238b come into contact with the cover member 100 in a state where the cover member 100 is inserted into the gap g2. Therefore, the cover member 100 is hardly removed from the gap g2.

Note that the number, positions, cross-sectional shapes, and the like of the projections disposed on the securing member 230B are not particularly limited. Furthermore, a projection can also be disposed on the securing member 230 including the claw portions 233 and 234 having the structure described in the first embodiment. Furthermore, a projection can also be disposed only on the main body 231 or only on a claw portion.

### <Third Modification>

Fig. 31 is a side view of a securing member 230C according to a third modification.

A plurality of protruding portions 239 are disposed on the main body 231 of the securing member 230C according to the third modification. The plurality of protruding portions 239 function as anti-slip portions when an operator grips the main body 231 with fingers or the like. Therefore, the operator can more smoothly perform work of securing the securing member 230C to the cover member 100. Note that the main body 231 in the securing member 230C may be made of a material that is not slippery. Even when the securing member 230C is configured as described above, effects similar to those described above can be exhibited.

### <Fourth Modification>

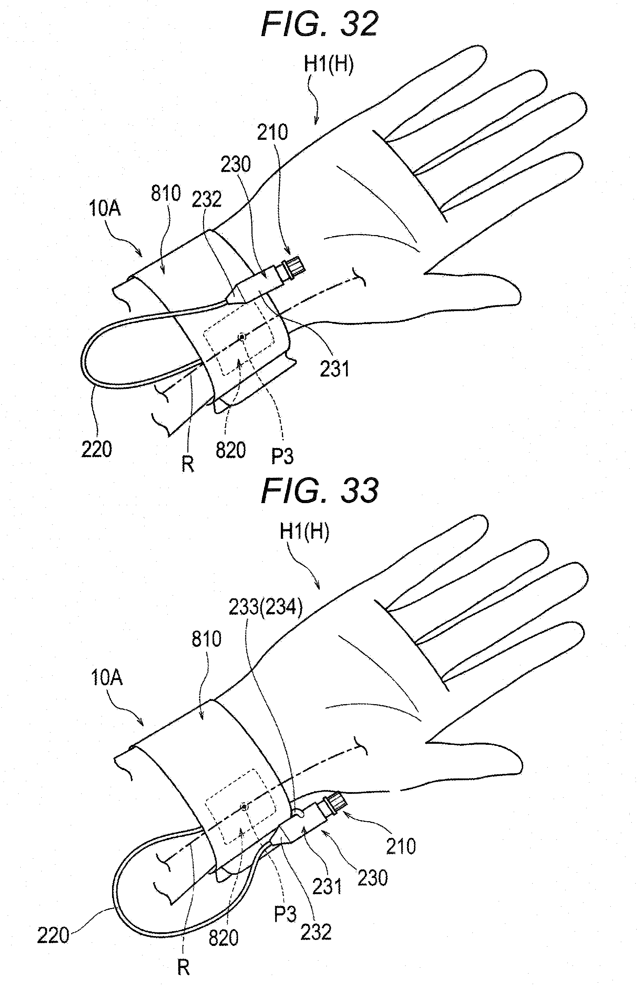

As illustrated in Figs. 32 and 33, the securing member 230 can also be applied to, for example, a hemostatic device 10A configured to be worn on an arm of a patient.

The hemostatic device 10A includes a cover member 810 formed in a band body shape and configured to be securable in a state of being wrapped around the arm of the patient, and an inflatable member 820 disposed on the cover member 810. The tube member 220 is coupled to the inflatable member 820. The tube member 220 is coupled to the main body 231 of the securing member 230. When hemostasis is performed using the hemostatic device 10A, for example, as illustrated in Figs. 32 and 33, the securing member 230 can be secured at a predetermined position of the cover member 810 by inserting the cover member 810 into the gap g2 (see Figs. 17 and 18).

Note that each of the hemostatic devices of the first to fourth modifications described above includes the inclined portion 232 provided in the main body of the securing member (see Figs. 28, 30, 31, and 32), which is similar to the hemostatic device 10 according to the embodiment described above. The inclined portion 232 included in each of the modifications is inclined toward the claw portion side. Furthermore, at least a part of the inclined portion 232 is configured to be located at a position on the proximal side of the distal end of the claw portion in the main body.

### <Second Embodiment>

Next, a securing member according to a second embodiment of the present invention will be described. In the description of the second embodiment, the description regarding the members and the process of using the hemostatic device already described in the first embodiment will be appropriately omitted. Furthermore, contents that are not particularly described in the second embodiment can be the same as those in the first embodiment.

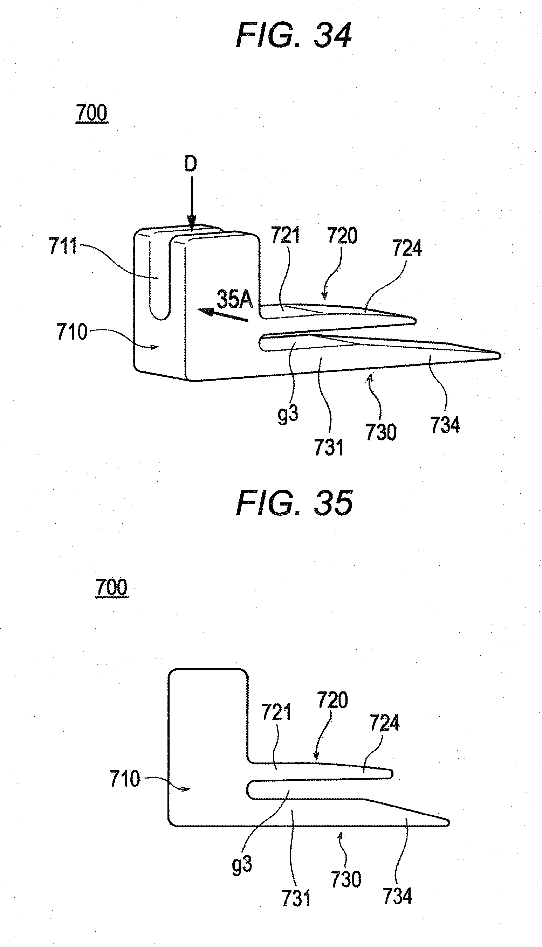

Figs. 34, 35, and 36 illustrate a securing member 700 according to the second embodiment.

The securing member 700 according to the second embodiment is configured to be detachably attached to the tube member 220.

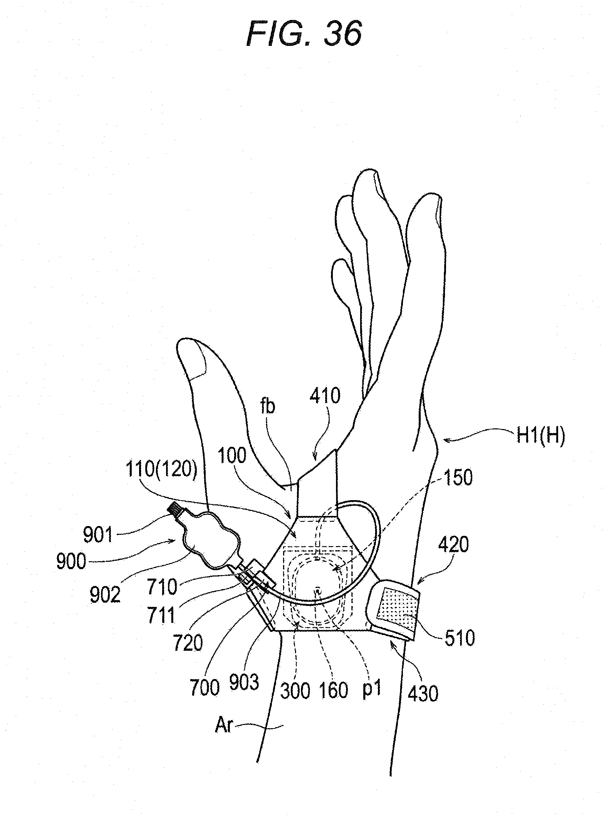

As illustrated in Fig. 36, a hemostatic device according to the second embodiment includes the cover member 100 configured to cover the first puncture site p1, the inflatable member 150 connected to the cover member 100 and configured to compress the first puncture site p1, an injection member 900 connected to the inflatable member 150 and configured to be capable of injecting a fluid into the inflatable member 150, and the securing member 700 configured to be capable of securing the injection member 900 to the cover member 100.

The injection member 900 includes a connector portion 901 and a tube member 903 connecting the connector portion 901 and the inflatable member 150.

A buffer member 902 having an inflatable space is disposed between the tube member 903 and the connector portion 901. The buffer member 902 is formed of a flexible bag-shaped member in which a space is formed. Note that the buffer member 902 may be provided with an arrow-shaped marker indicating an insertion direction of a syringe into the connector portion 901. When air is injected into the inner cavity 150a of the inflatable member 150, the inflatable member 150 inflates. When the inflatable member 150 inflates, the buffer member 902 communicating with the inner cavity 150a of the inflatable member 150 via the tube member 903 expands. An operator can grasp that the inflatable member 150 inflates without leakage of air by visually confirming the expansion of the buffer member 902.

As illustrated in Figs. 34 and 35, the securing member 700 includes a main body 710 in which a groove 711 capable of securing the tube member 903 is formed, a first claw portion 720 extending from the main body 710, and a second claw portion 730 extending in parallel with the first claw portion 720 and forming a gap g3 into which the cover member 100 can be inserted, the gap being formed between the first claw portion 720 and the second claw portion 730.

The securing member 700 can connect the tube member 903 to the main body 710 by pushing the tube member 903 into the groove 711 along a direction indicated by an arrow D in Fig. 34.

The securing member 700 can be secured to the cover member 100 by inserting the cover member 100 into the gap g3 between the first claw portion 720 and the second claw portion 730 in the securing member 700.

The first claw portion 720 has a root portion 721 connected to the main body 710 and a tapered portion 724 extending to a distal side of the root portion 721. The tapered portion 724 is configured to have a thickness gradually decreasing toward a distal side of the first claw portion 720.

The second claw portion 730 has a root portion 731 connected to the main body 710 and a tapered portion 734 extending to a distal side of the root portion 731. The tapered portion 734 is configured to have a thickness gradually decreasing toward a distal side of the second claw portion 730. A distal end of the tapered portion 734 of the second claw portion 730 is disposed at a position farther away from the main body 710 than a distal end of the tapered portion 724 of the first claw portion 720. That is, the second claw portion 730 is longer in an extending direction than the first claw portion 720.

When the cover member 100 is inserted into the gap g3, the securing member 700 is guided to be inserted into the gap g3 along the tapered portion 734 of the second claw portion 730. Therefore, an operator can smoothly insert the cover member 100 into the gap g3.

The securing member 700 is connected to the hemostatic device via the tube member 903 by fitting the tube member 903 into the groove 711. Therefore, the securing member 230 is separated from the hemostatic device in a state where the tube member 903 is not fitted into the groove 711. The securing member 700 can be packed together with the hemostatic device in the state of being separated from the hemostatic device in a state before the hemostatic device is used (in a state of being transported or stored). The operator can connect the tube member 903 to the securing member 700 when starting hemostasis using the hemostatic device. As illustrated in Fig. 36, the operator can secure the securing member 700 connected to the tube member 903 to any position of the cover member 100.

By securing the securing member 700 to the cover member 100, the operator can prevent the tube member 903 from moving following a movement of the right hand H1 of a patient in a state where the hemostatic device is worn on the right hand H1 of the patient. Furthermore, when the securing member 700 is connected to the cover member 100, the securing member 700 can be secured to the cover member 100 by bringing the securing member 700 close to the cover member 100 along a direction substantially parallel with a plane direction of the cover member 100. Therefore, when the securing member 700 is secured to the cover member 100, it is possible to prevent a force in a direction of pressing the inflatable member 150 against a body surface of the right hand H1 of the patient from being applied to the hemostatic device 10.

Although the hemostatic device according to the present invention has been described above through the plurality of embodiments and modifications, the present invention is not limited only to the content described in the specification and can be appropriately changed based on the description of the claims.

The shape, size, and the like of each part of the hemostatic device are not particularly limited as long as a puncture site can be compressed to stop bleeding by the inflatable member disposed at the puncture site, and can be appropriately changed.

Specific shapes (planar shape and cross-sectional shape) of the inflatable member and the support member are not limited to the shapes illustrated in the embodiments.

For example, the securing member can be partially or entirely made of a flexible material in order to reduce a load on the patient's body wearing the hemostatic device or to improve contact with skin at the time of being worn. Furthermore, the securing member may be configured to include a clip structure that enables a distance between the claw portion and the main body to be variable using a resilient force or the like of an elastic member. When the securing member is configured to have the clip structure, the securing member and the cover member can be easily secured and released by opening and closing the clip.

The present application is based on Japanese Patent Application No. 2022-44655 filed on March 18, 2022, the disclosure content of which is incorporated herein by reference in its entirety.

### Reference Signs List

10 Hemostatic device
10A Hemostatic device
100 Cover member
110 Main body
120 Sheet material
150 Inflatable member
150a Inner cavity of inflatable member
200 Injection member
210 Connector portion
211 Port portion
213 Valve portion
220 Tube member
230 Securing member
231 Main body of securing member
232 Inclined portion
233 First claw portion (claw portion)
233a Distal end of first claw portion
234 Second claw portion (claw portion)
234a Distal end of second claw portion
236 Coupling portion
237 Coupling portion
230A Securing member
233A Claw portion
233b Flat portion
230B Securing member
238a First projection (projection)
238b Second projection (projection)
230C Securing member
239 Protruding portion
300 Support member
410 First band body
420 Second band body
430 Third band body
600 Introducer
700 Securing member
710 Main body of securing member
711 Groove
720 First claw portion
730 Second claw portion
900 Injection member
901 Connector portion
902 Buffer member
903 Tube member
g2 Gap between main body and claw portion
g3 Gap between main body and claw portion
w3 Width of claw portion
w4 Width of main body of securing member
H Hand
H1 Right hand
Ar Forearm
fb Interdigital part
Sb Snuff box
B1 Metacarpal bone of index finger
B2 Metacarpal bone of thumb
T1 Extensor pollicis longus muscle
T2 Extensor pollicis brevis muscle
V1 Blood vessel (distal radial artery)
V2 Artery located at snuff box
p1 First puncture site (puncture site)
p2 Second puncture site (puncture site)

## Claims

1. A hemostatic device comprising:
a cover member configured to cover a puncture site formed on a patient;
an inflatable member connected to the cover member and configured to compress the puncture site;
an injection member connected to the inflatable member and configured to be capable of injecting a fluid into the inflatable member; and
a securing member configured to be capable of securing the injection member to the cover member, wherein
the injection member includes a connector portion and a tube member coupled to the inflatable member,
the securing member includes a main body connecting the connector portion and the tube member, a claw portion extending in parallel with the main body toward the inflatable member, and a coupling portion configured to be capable of inserting the cover member between the main body and the claw portion and coupling the main body and the claw portion, and
a width of the claw portion is smaller than a width of the main body at a position where the claw portion overlaps the main body in a plan view.

2. The hemostatic device according to claim 1, wherein
the main body includes an inclined portion inclined toward the claw portion, and
the inclined portion extends from a position on a distal side of a distal end of the claw portion to a position on a proximal side of the distal end of the claw portion.

3. The hemostatic device according to claim 1 or 2, wherein the main body has a circular or elliptical cross-sectional shape.

4. The hemostatic device according to any one of claims 1 to 3, wherein
the claw portion includes a flat portion configured to maintain a part of the cover member in a flat shape in a state where the cover member is inserted between the main body and the claw portion, and
the flat portion faces the main body across a gap formed between the main body and the claw portion.

5. The hemostatic device according to any one of claims 1 to 4, wherein the coupling portion is curved from the main body toward a distal side of the claw portion.

6. The hemostatic device according to any one of claims 1 to 5, wherein the main body and/or the claw portion has a projection protruding toward a gap formed between the main body and the claw portion.

7. The hemostatic device according to any one of claims 1 to 6, wherein
the cover member includes a main body where the inflatable member is located, a first band body that is configured to be disposed between fingers of the patient and extends in a first direction from the main body of the cover member, a second band body extending in a second direction different from the first direction from the main body of the cover member, and a third band body facing the second band body across the inflatable member and extending in a different direction from the first band body and the second band body from the main body of the cover member, and
the claw portion is configured to cause any one of the main body of the cover member, the first band body, the second band body, and the third band body to be inserted between the claw portion and the coupling portion.

8. A hemostatic device comprising:
a cover member configured to cover a puncture site formed on a patient;
an inflatable member connected to the cover member and configured to compress the puncture site;
an injection member connected to the inflatable member and configured to be capable of injecting a fluid into the inflatable member; and
a securing member configured to be capable of securing the injection member to the cover member, wherein
the injection member includes a connector portion and a tube member connecting the connector portion and the inflatable member, and
the securing member includes a main body in which a groove capable of securing the tube member is formed, a first claw portion extending from the main body, and a second claw portion extending in parallel with the first claw portion and forming a gap into which the cover member is insertable, the gap being formed between the first claw portion and the second claw portion.

9. A hemostatic device comprising:
a cover member configured to cover a puncture site formed on a patient;
an inflatable member connected to the cover member and configured to compress the puncture site;
an injection member connected to the inflatable member and configured to be capable of injecting a fluid into the inflatable member; and
a securing member configured to be capable of securing the injection member to the cover member, wherein
the injection member includes a connector portion and a tube member coupled to the inflatable member,
the securing member includes a main body connecting the connector portion and the tube member, a claw portion extending in parallel with the main body toward the inflatable member, and a coupling portion configured to be capable of inserting the cover member between the main body and the claw portion and coupling the main body and the claw portion,
the main body includes an inclined portion inclined toward the claw portion, and
at least a part of the inclined portion is configured to be located at a position on a proximal side of a distal end of the claw portion in the main body.

10. The hemostatic device according to claim 9, wherein
the claw portion includes a flat portion configured to maintain a part of the cover member in a flat shape in a state where the cover member is inserted between the main body and the claw portion, and
the flat portion faces the main body across a gap formed between the main body and the claw portion.

11. The hemostatic device according to claim 9 or 10, wherein a width of the claw portion is smaller than a width of the main body at a position where the claw portion overlaps the main body in a plan view.
